# EUROPEAN PATENT SPECIFICATION

(11) **EP 1 553 962 B1**
(45) Date of publication and mention of the grant of the patent: **24.02.2010**
(21) Application number: 03731504.1
(22) Date of filing: 02.06.2003
(51) Int. Cl.: C07K 14/435, A61K 38/17, A61K 31/175, A61K 31/4188, A61P 35/00

(54) **COMBINATION CHEMOTHERAPY WITH CHLOROTOXIN**
KOMBINATIONSCHEMOTHERAPIE MIT CHLOROTOXIN
CHIMIOTHERAPIE COMBINEE COMPRENANT LA CHLOROTOXINE

(30) Priority: 31.05.2002 US 384171 P; 27.08.2002 US 406033 P
(43) Date of publication of application: 20.07.2005
(60) Divisional application: 09167197.4
(73) Proprietor: Transmolecular, Inc., Birmingham, AL 35243 (US)
(72) Inventor: ALVAREZ, Vermont L., Birmingham, AL 35243 (US); GRIMES, Carol A., Birmingham, AL 35243 (US); GONDA, Matthew A., Birmingham, AL 35243 (US)
(74) Representative: Lee, Nicholas John
(86) International application number: PCT/US2003/017410
(87) International publication number: WO 2003/101474

(56) References cited:
- US-A- 5 905 027
- US-A- 6 028 174
- US-A1- 2002 065 216
- US-B1- 6 319 891
- DATABASE UniProt [Online] entry version 41 10 May 2002 (2002-05-10), "Chlorotoxin (CTX). 1 36 Chlorotoxin." XP002448248 retrieved from EBI accession no. UNIPROT:P45639 Database accession no. P45639
- DEBIN J A ET AL: "PURIFICATION AND CHARACTERIZATION OF CHLOROTOXIN, A CHLORIDE CHANNEL LIGAND FROM THE VENOM OF THE SCORPION" AMERICAN JOURNAL OF PHYSIOLOGY, AMERICAN PHYSIOLOGICAL SOCIETY, BETHESDA, MD, US, vol. 2, no. 1, February 1993 (1993-02), pages C361-C369, XP008055160 ISSN: 0002-9513
- MALINOWSKA D H ET AL: "Recombinant chlorotoxin: An inhibitor of gastric Cl-channels" BIOPHYSICAL JOURNAL, vol. 66, no. 2 PART 2, 1994, page A100, XP002448245 & THIRTY-EIGHTH ANNUAL MEETING OF THE BIOPHYSICAL SOCIETY; NEW ORLEANS, LOUISIANA, USA; MARCH 6-10, 1994 ISSN: 0006-3495
- LIPPENS G ET AL: "NMR Sequencial Assignments and Solution Structure of Chlorotoxin, a Small scorpion Toxin that Blocks Chloride Channels" BIOCHEMISTRY, AMERICAN CHEMICAL SOCIETY. EASTON, PA, US, vol. 34, no. 1, 1995, pages 13-21, XP002995989 ISSN: 0006-2960
- ULLRICH NICOLE ET AL: "Human astrocytoma cells express a unique chloride current" NEUROREPORT, vol. 7, no. 5, 1996, pages 1020-1024, XP009088599 ISSN: 0959-4965
- ULLRICH NICOLE ET AL: "Biophysical and pharmacological characterization of chloride currents in human astrocytoma cells" AMERICAN JOURNAL OF PHYSIOLOGY, vol. 270, no. 5 PART 1, 1996, pages C1511-C1521, XP009088598 ISSN: 0002-9513
- SOROCEANU L ET AL: "USE OF CHLOROTOXIN FOR TARGETING OF PRIMARY BRAIN TUMORS" CANCER RESEARCH, AMERICAN ASSOCIATION FOR CANCER RESEARCH, BALTIMORE, MD, US, vol. 58, no. 21, 1 November 1998 (1998-11-01), pages 4871-4879, XP001146137 ISSN: 0008-5472
- SOROCEANU L ET AL: "Modulation of glioma cell migration and invasion using Cl- and K+ iron channel blockers" JOURNAL OF NEUROSCIENCE, NEW YORK, NY, US, vol. 19, no. 14, 15 July 1999 (1999-07-15), pages 5942-5954, XP002964295 ISSN: 0270-6474
- LYONS S A ET AL: "CHLOROTOXIN, A SCORPION-DERIVED PEPTIDE, SPECIFICALLY BINDS TO GLIOMAS AND TUMORS OF NEUROECTODERMAL ORIGIN" GLIA, WILEY-LISS, NEW YORK, NY, US, vol. 39, no. 2, 29 May 2002 (2002-05-29), pages 162-173, XP009006886 ISSN: 0894-1491
- DESHANE JESSY ET AL: "Chlorotoxin inhibits glioma cell invasion via matrix metalloproteinase-2" JOURNAL OF BIOLOGICAL CHEMISTRY, AMERICAN SOCIETY OF BIOLOCHEMICAL BIOLOGISTS, BIRMINGHAM,, US, vol. 278, no. 6, 25 November 2002 (2002-11-25), pages 4135-4144, XP002398052 ISSN: 0021-9258
- NEWLANDS E S ET AL: "TEMOZOLOMIDE: A REVIEW OF ITS DISCOVERY, CHEMICAL PROPERTIES, PRE-CLINICAL DEVELOPMENT AND CLINICAL TRIALS" CANCER TREATMENT REVIEWS, SAUNDERS, US, vol. 23, no. 1, 1997, pages 35-61, XP000921344 ISSN: 0305-7372
- DATABASE MEDLINE [Online] US NATIONAL LIBRARY OF MEDICINE (NLM), BETHESDA, MD, US; June 1992 (1992-06), LEVIN V A: "The place of hydroxyurea in the treatment of primary brain tumors." XP002448249 Database accession no. NLM1641655 & SEMINARS IN ONCOLOGY JUN 1992, vol. 19, no. 3 Suppl 9, June 1992 (1992-06), pages 34-39, ISSN: 0093-7754

### SEQUENCE LISTING

<110> ALVAREZ, Vernon L.
   GONDA, Matthew A.
<120> Treatment of Cell Proliferative Disorders with Chlorotoxin
<130> 2006636-0018
<140> EP03731504.1
   <141> 2002-05-31
<150> US 60/406,033
   <151> 2002-08-27
<150> US 60/384,171
   <151> 2002-05-31
<160> 95
<170> PatentIn version 3.5
<210> 1
   <211> 36
   <212> PRT
   <213> Leiurus quinquestriatus
<220>
   <221> misc_feature
   <223> Chlorotoxin
<400> 1
   Met Cys Met Pro Cys Phe Thr Thr Asp His Gln Met Ala Arg Lys Cys
   1 5 10 15
   Asp Asp Cys Cys Gly Gly Lys Gly Arg Gly Lys Cys Tyr Gly Pro Gln
   20 25 30
   Cys Leu Cys Arg
   35
<210> 2
   <211> 42
   <212> PRT
   <213> Leiurus quinquestriatus
<400> 2
   His His His His His His Met Cys Met Pro Cys Phe Thr Thr Asp His
   1 5 10 15
   Gln Met Ala Arg Lys Cys Asp Asp Cys Cys Gly Gly Lys Gly Arg Gly
   20 25 30
   Lys Cys Tyr Gly Pro Gln Cys Leu Cys Arg
   35 40
<210> 3
   <211> 37
   <212> PRT
   <213> Leiurus quinquestriatus
<400> 3
   Tyr Met Cys Met Pro Cys Phe Thr Thr Asp His Gln Met Ala Arg Lys
   1 5 10 15
   Cys Asp Asp Cys Cys Gly Gly Lys Gly Arg Gly Lys Cys Tyr Gly Pro
   20 25 30
   Gln Cys Leu Cys Arg
   35
<210> 4
   <211> 39
   <212> PRT
   <213> Leiurus quinquestriatus
<400> 4
   Tyr Ser Tyr Met Cys Met Pro Cys Phe Thr Thr Asp His Gln Met Ala
   1 5 10 15
   Arg Lys Cys Asp Asp Cys Cys Gly Gly Lys Gly Arg Gly Lys Cys Tyr
   20 25 30
   Gly Pro Gln Cys Leu Cys Arg
   35
<210> 5
   <211> 36
   <212> PRT
   <213> Artificial sequence
<220>
   <223> Chlorotoxin variant
<400> 5
   Met Cys Met Pro Cys Phe Thr Thr Asp His Gln Met Ala Arg Lys Cys
   1 5 10 15
   Asp Asp Cys Cys Gly Gly Lys Gly Arg Gly Lys Cys Phe Gly Pro Gln
   20 25 30
   Cys Leu Cys Arg
   35
<210> 6
   <211> 35
   <212> PRT
   <213> Artificial sequence
<220>
   <223> Chlorotoxin variant
<400> 6
   Arg Cys Lys Pro Cys Phe Thr Thr Asp Pro Gln Met Ser Lys Lys Cys
   1 5 10 15
   Ala Asp Cys Cys Gly Gly Lys Gly Lys Gly Lys Cys Tyr Gly Pro Gln
   20 25 30
   Cys Leu Cys
   35
<210> 7
   <211> 38
   <212> PRT
   <213> Artificial sequence
<220>
   <223> Chlorotoxin variant
<400> 7
   Arg Cys Ser Pro Cys Phe Thr Thr Asp Gln Gln Met Thr Lys Lys Cys
   1 5 10 15
   Tyr Asp Cys Cys Gly Gly Lys Gly Lys Gly Lys Cys Tyr Gly Pro Gln
   20 25 30
   Cys Ile Cys Ala Pro Tyr
   35
<210> 8
   <211> 7
   <212> PRT
   <213> Leiurus quinquestriatus
<220>
   <221> misc_feature
   <223> Derivative of Chlorotoxin: amino acid residues 23-29
<400> 8
   Lys Gly Arg Gly Lys Ser Tyr
   1 5
<210> 9
   <211> 7
   <212> PRT
   <213> Leiurus quinquestriatus
<220>
   <221> misc_feature
   <223> Derivative of Chlorotoxin: amino acid residues 8-14
<400> 9
   Thr Asp His Gln Met Ala Arg
   1 5
<210> 10
   <211> 9
   <212> PRT
   <213> Artificial sequence
<220>
   <223> Chlorotoxin alpha peptide
<400> 10
   Thr Asp His Gln Met Ala Arg Lys Ser
   1 5
<210> 11
   <211> 9
   <212> PRT
   <213> Artificial sequence
<220>
   <223> Variant of chlorotoxin alpha peptide
<400> 11
   Thr Ala His Ala Met Ala Arg Lys Ser
   1 5
<210> 12
   <211> 36
   <212> PRT
   <213> Artificial sequence
<220>
   <223> Variant peptide of chlorotoxin
<400> 12
   Met Cys Met Pro Cys Phe Thr Thr Ala His Ala Met Ala Arg Lys Cys
   1 5 10 15
   Asp Asp Cys Cys Gly Gly Lys Gly Arg Cys Lys Cys Tyr Gly Pro Gln
   20 25 30
   Cys Leu Cys Arg
   35
<210> 13
   <211> 9
   <212> PRT
   <213> Artificial
<220>
   <223> motif for chlorotoxin derivatives
<220>
   <221> Variant
   <222> (3)..(3)
   <223> Xaa = Asp or Glu
<220>
   <221> Variant
   <222> (4)..(4)
   <223> Xaa = Ala, Arg, Asn, Asp, Cys, Gln, Glu, Gly, His, Ile, Leu, Lys, Met, Phe, Pro, Ser, Thr, Trp, Tyr or Val
<220>
   <221> Variant
   <222> (5)..(5)
   <223> Xaa = Asn or Gln
<220>
   <221> Variant
   <222> (7)..(7)
   <223> Xaa = Ser, Thr, or Ala
<220>
   <221> Variant
   <222> (8)..(8)
   <223> Xaa = His, Lys, or Arg
<400> 13
   Thr Thr Xaa Xaa Xaa Met Xaa Xaa Lys
   1 5
<210> 14
   <211> 9
   <212> PRT
   <213> Leiurus quinquestriatus
<400> 14
   Thr Thr Asp His Gln Met Ala Arg Lys
   1 5
<210> 15
   <211> 35
   <212> PRT
   <213> Mesobuthus tamulus
<400> 15
   Arg Cys Lys Pro Cys Phe Thr Thr Asp Pro Gln Met Ser Lys Lys Cys
   1 5 10 15
   Ala Asp Cys Cys Gly Gly Lys Gly Lys Gly Lys Cys Tyr Gly Pro Gln
   20 25 30
   Cys Leu Cys
   35
<210> 16
   <211> 34
   <212> PRT
   <213> Artificial sequence
<220>
   <223> Small Toxin consensus sequence
<220>
   <221> MISC_FEATURE
   <222> (2)..(2)
   <223> Xaa can be Met or Lys
<220>
   <221> MISC_FEATURE
   <222> (9)..(9)
   <223> Xaa can be His or Pro
<220>
   <221> MISC_FEATURE
   <222> (16)..(16)
   <223> Xaa can be Asp or Ala
<400> 16
   Cys Xaa Pro Cys Phe Thr Thr Asp Xaa Gln Met Ala Lys Lys Cys Xaa
   1 5 10 15
   Asp Cys Cys Gly Gly Lys Gly Lys Gly Lys Cys Tyr Gly Pro Gln Cys
   20 25 30
   Leu Cys
<210> 17
   <211> 38
   <212> PRT
   <213> Leiurus quinquestriatus
<400> 17
   Arg Cys Ser Pro Cys Phe Thr Thr Asp Gln Gln Met Thr Lys Lys Cys
   1 5 10 15
   Tyr Asp Cys Cys Gly Gly Lys Gly Lys Gly Lys Cys Tyr Gly Pro Gln
   20 25 30
   Cys Ile Cys Ala Pro Tyr
   35
<210> 18
   <211> 34
   <212> PRT
   <213> Artificial sequence
<220>
   <223> Probable Toxin LQH 8/6 consensus sequence
<220>
   <221> MISC_FEATURE
   <222> (2)..(2)
   <223> Xaa can be Met or Ser
<220>
   <221> MISC_FEATURE
   <222> (9)..(9)
   <223> Xaa can be His or Gln
<220>
   <221> MISC_FEATURE
   <222> (12)..(12)
   <223> Xaa can be Ala or Thr
<220>
   <221> MISC_FEATURE
   <222> (16)..(16)
   <223> Xaa can be Asp or Tyr
<400> 18
   Cys Xaa Pro Cys Phe Thr Thr Asp Xaa Gln Met Xaa Lys Lys Cys Xaa
   1 5 10 15
   Asp Cys Cys Gly Gly Lys Gly Lys Gly Lys Cys Tyr Gly Pro Gln Cys
   20 25 30
   Ile Cys
<210> 19
   <211> 61
   <212> PRT
   <213> Mesobuthus martensii
<220>
   <221> MISC_FEATURE
   <222> (1)..(61)
   <223> Xaa can be any amino acid
<400> 19
   Met Lys Phe Leu Tyr Gly Ile Val Phe Ile Ala Leu Phe Leu Thr Val
   1 5 10 15
   Met Phe Ala Thr Gln Thr Asp Gly Cys Gly Pro Cys Phe Thr Thr Asp
   20 25 30
   Ala Asn Met Ala Arg Lys Cys Arg Glu Cys Cys Gly Gly Ile Gly Xaa
   35 40 45
   Xaa Lys Cys Phe Gly Pro Gln Cys Leu Cys Asn Arg Ile
   50 55 60
<210> 20
   <211> 34
   <212> PRT
   <213> Artificial sequence
<220>
   <223> Chinese Scorpion consensus sequence
<220>
   <221> MISC_FEATURE
   <222> (2)..(2)
   <223> Xaa can be Met or Gly
<220>
   <221> MISC_FEATURE
   <222> (9)..(9)
   <223> Xaa can be His or Ala
<220>
   <221> MISC_FEATURE
   <222> (16)..(16)
   <223> Xaa can be Asp or Arg
<220>
   <221> MISC_FEATURE
   <222> (22)..(22)
   <223> Xaa can be Lys or Ile
<220>
   <221> MISC_FEATURE
   <222> (24)..(25)
   <223> Xaa can be any amino acid
<400> 20
   Cys Xaa Pro Cys Phe Thr Thr Asp Xaa Asn Met Ala Arg Lys Cys Xaa
   1 5 10 15
   Asp Cys Cys Gly Gly Xaa Gly Xaa Xaa Lys Cys Phe Gly Pro Gln Cys
   20 25 30
   Leu Cys
<210> 21
   <211> 59
   <212> PRT
   <213> Mesobuthus martensii
<400> 21
   Met Lys Phe Leu Tyr Gly Ile Val Phe Ile Ala Leu Phe Leu Thr Val
   1 5 10 15
   Met Phe Ala Thr Gln Thr Asp Gly Cys Gly Pro Cys Phe Thr Thr Asp
   20 25 30
   Ala Asn Met Ala Arg Lys Cys Arg Glu Cys Cys Gly Gly Ile Gly Lys
   35 40 45
   Cys Phe Gly Pro Gln Cys Leu Cys Asn Arg Ile
   50 55
<210> 22
   <211> 32
   <212> PRT
   <213> Artificial sequence
<220>
   <223> Chinese Scorpion consensus sequence
<220>
   <221> MISC_FEATURE
   <222> (2)..(2)
   <223> Xaa can be Met or Gly
<220>
   <221> MISC_FEATURE
   <222> (9)..(9)
   <223> Xaa can be His or Ala
<220>
   <221> MISC_FEATURE
   <222> (16)..(16)
   <223> Xaa can be Asp or Arg
<220>
   <221> MISC_FEATURE
   <222> (22)..(22)
   <223> Xaa can be Lys or Ile
<220>
   <221> MISC_FEATURE
   <222> (25)..(25)
   <223> Xaa can be Gly or Cys
<220>
   <221> MISC_FEATURE
   <222> (26)..(26)
   <223> Xaa can be Lys or Phe
<220>
   <221> MISC_FEATURE
   <222> (27)..(27)
   <223> Xaa can be Cys or Gly
<220>
   <221> MISC_FEATURE
   <222> (28)..(28)
   <223> Xaa can be Tyr or Pro
<220>
   <221> MISC_FEATURE
   <222> (29)..(29)
   <223> Xaa can be Gly or Gln
<220>
   <221> MISC_FEATURE
   <222> (30)..(30)
   <223> Xaa can be Pro or Cys
<220>
   <221> MISC_FEATURE
   <222> (31)..(31)
   <223> Xaa can be Gln or Leu
<400> 22
   Cys Xaa Pro Cys Phe Thr Thr Asp Xaa Asn Met Ala Arg Lys Cys Xaa
   1 5 10 15
   Asp Cys Cys Gly Gly Xaa Gly Lys Xaa Xaa Xaa Xaa Xaa Xaa Xaa Cys
   20 25 30
<210> 23
   <211> 37
   <212> PRT
   <213> Mesobuthus eupeus
<220>
   <221> MISC_FEATURE
   <222> (1)..(37)
   <223> Xaa can be any amino acid
<400> 23
   Met Cys Met Pro Cys Phe Thr Thr Asp Pro Asn Met Ala Asn Lys Cys
   1 5 10 15
   Arg Asp Cys Cys Gly Gly Xaa Gly Lys Xaa Lys Cys Phe Gly Pro Gln
   20 25 30
   Cys Leu Cys Asn Arg
   35
<210> 24
   <211> 35
   <212> PRT
   <213> Artificial sequence
<220>
   <223> Insect toxin I5 consensus sequence
<220>
   <221> MISC_FEATURE
   <222> (10)..(10)
   <223> Xaa can be His or Pro
<220>
   <221> MISC_FEATURE
   <222> (14)..(14)
   <223> Xaa can be Arg or Asn
<220>
   <221> MISC_FEATURE
   <222> (17)..(17)
   <223> Xaa can be Asp or Arg
<220>
   <221> MISC_FEATURE
   <222> (23)..(26)
   <223> Xaa can be any amino acid
<400> 24
   Met Cys Met Pro Cys Phe Thr Thr Asp Xaa Asn Met Ala Xaa Lys Cys
   1 5 10 15
   Xaa Asp Cys Cys Gly Gly Xaa Gly Lys Xaa Lys Cys Phe Gly Pro Gln
   20 25 30
   Cys Leu Cys
   35
<210> 25
   <211> 35
   <212> PRT
   <213> Mesobuthus eupeus
<400> 25
   Met Cys Met Pro Cys Phe Thr Thr Asp Pro Asn Met Ala Asn Lys Cys
   1 5 10 15
   Arg Asp Cys Cys Gly Gly Gly Lys Lys Cys Phe Gly Pro Gln Cys Leu
   20 25 30
   Cys Asn Arg
   35
<210> 26
   <211> 33
   <212> PRT
   <213> Artificial sequence
<220>
   <223> Insect toxin I5 consensus sequence
<220>
   <221> MISC_FEATURE
   <222> (10)..(10)
   <223> Xaa can be His or Pro
<220>
   <221> MISC_FEATURE
   <222> (14)..(14)
   <223> Xaa can be Arg or Asn
<220>
   <221> MISC_FEATURE
   <222> (17)..(17)
   <223> Xaa can be Asp or Arg
<220>
   <221> MISC_FEATURE
   <222> (23)..(24)
   <223> Xaa can be Lys or Gly
<220>
   <221> MISC_FEATURE
   <222> (26)..(26)
   <223> Xaa can be Gly or Cys
<220>
   <221> MISC_FEATURE
   <222> (27)..(27)
   <223> Xaa can be Lys or Phe
<220>
   <221> MISC_FEATURE
   <222> (28)..(28)
   <223> Xaa can be Cys or Gly
<220>
   <221> MISC_FEATURE
   <222> (29)..(29)
   <223> Xaa can be Tyr or Pro
<220>
   <221> MISC_FEATURE
   <222> (30)..(30)
   <223> Xaa can be Gly or Gln
<220>
   <221> MISC_FEATURE
   <222> (31)..(31)
   <223> Xaa can be Pro or Cys
<220>
   <221> MISC_FEATURE
   <222> (32)..(32)
   <223> Xaa can be Gln or Leu
<400> 26
   Met Cys Met Pro Cys Phe Thr Thr Asp Xaa Asn Met Ala Xaa Lys Cys
   1 5 10 15
   Xaa Asp Cys Cys Gly Gly Xaa Xaa Lys Xaa Xaa Xaa Xaa Xaa Xaa Xaa
   20 25 30
   Cys
<210> 27
   <211> 38
   <212> PRT
   <213> Mesobuthus eupeus
<220>
   <221> MISC_FEATURE
   <222> (1)..(38)
   <223> Xaa can be any amino acid
<400> 27
   Met Cys Met Pro Cys Phe Thr Thr Arg Pro Asp Met Ala Gln Gln Cys
   1 5 10 15
   Arg Ala Cys Cys Lys Gly Xaa Xaa Arg Gly Lys Cys Phe Gly Pro Gln
   20 25 30
   Cys Leu Cys Gly Tyr Asp
   35
<210> 28
   <211> 35
   <212> PRT
   <213> Artificial sequence
<220>
   <223> Insetotoxin I1 consensus sequence
<220>
   <221> MISC_FEATURE
   <222> (9)..(9)
   <223> Xaa can be Asp or Arg
<220>
   <221> MISC_FEATURE
   <222> (10)..(10)
   <223> Xaa can be His or Pro
<220>
   <221> MISC_FEATURE
   <222> (11)..(11)
   <223> Xaa can be Gln or Asp
<220>
   <221> MISC_FEATURE
   <222> (14)..(14)
   <223> Xaa can be Arg or Gln
<220>
   <221> MISC_FEATURE
   <222> (15)..(15)
   <223> Xaa can be Lys or Gln
<220>
   <221> MISC_FEATURE
   <222> (17)..(17)
   <223> Xaa can be Asp or Arg
<220>
   <221> MISC_FEATURE
   <222> (18)..(18)
   <223> Xaa can be Asp or Ala
<220>
   <221> MISC_FEATURE
   <222> (21)..(21)
   <223> Xaa can be Gly or Lys
<220>
   <221> MISC_FEATURE
   <222> (23)..(24)
   <223> Xaa can be any amino acid
<400> 28
   Met Cys Met Pro Cys Phe Thr Thr Xaa Xaa Xaa Met Ala Xaa Xaa Cys
   1 5 10 15
   Xaa Xaa Cys Cys Xaa Gly Xaa Xaa Arg Gly Lys Cys Phe Gly Pro Gln
   20 25 30
   Cys Leu Cys
   35
<210> 29
   <211> 36
   <212> PRT
   <213> Mesobuthus eupeus
<400> 29
   Met Cys Met Pro Cys Phe Thr Thr Arg Pro Asp Met Ala Gln Gln Cys
   1 5 10 15
   Arg Ala Cys Cys Lys Gly Arg Gly Lys Cys Phe Gly Pro Gln Cys Leu
   20 25 30
   Cys Gly Tyr Asp
   35
<210> 30
   <211> 33
   <212> PRT
   <213> Artificial sequence
<220>
   <223> Insectotoxin I1 consensus sequence
<220>
   <221> MISC_FEATURE
   <222> (9)..(9)
   <223> Xaa can be Asp or Arg
<220>
   <221> MISC_FEATURE
   <222> (10)..(10)
   <223> Xaa can be His or Pro
<220>
   <221> MISC_FEATURE
   <222> (11)..(11)
   <223> Xaa can be Gln or Asp
<220>
   <221> MISC_FEATURE
   <222> (14)..(14)
   <223> Xaa can be Arg or Gln
<220>
   <221> MISC_FEATURE
   <222> (15)..(15)
   <223> Xaa can be Lys or Gln
<220>
   <221> MISC_FEATURE
   <222> (17)..(17)
   <223> Xaa can be Asp or Arg
<220>
   <221> MISC_FEATURE
   <222> (18)..(18)
   <223> Xaa can be Asp or Arg
<220>
   <221> MISC_FEATURE
   <222> (21)..(21)
   <223> Xaa can be Gly or Lys
<220>
   <221> MISC_FEATURE
   <222> (26)..(26)
   <223> Xaa can be Gly or Cys
<220>
   <221> MISC_FEATURE
   <222> (27)..(27)
   <223> Xaa can be Lys or Phe
<220>
   <221> MISC_FEATURE
   <222> (28)..(28)
   <223> Xaa can be Cys or Gly
<220>
   <221> MISC_FEATURE
   <222> (29)..(29)
   <223> Xaa can be Tyr or Pro
<220>
   <221> MISC_FEATURE
   <222> (30)..(30)
   <223> Xaa can be Gly or Gln
<220>
   <221> MISC_FEATURE
   <222> (31)..(31)
   <223> Xaa can be Pro or Cys
<220>
   <221> MISC_FEATURE
   <222> (32)..(32)
   <223> Xaa can be Gln or Leu
<400> 30
   Met Cys Met Pro Cys Phe Thr Thr Xaa Xaa Xaa Met Ala Xaa Xaa Cys
   1               5                   10                  15
   Xaa Xaa Cys Cys Xaa Gly Lys Gly Lys Xaa Xaa Xaa Xaa Xaa Xaa Xaa
               20                  25                  30
   Cys
<210> 31
   <211> 37
   <212> PRT
   <213> Mesobuthus eupeus
<220>
   <221> MISC_FEATURE
   <222> (1)..(37)
   <223> Xaa can be any amino acid
<400> 31
   Met Cys Met Pro Cys Phe Thr Thr Asp Pro Asn Met Ala Lys Lys Cys
   1 5 10 15
   Arg Asp Cys Cys Gly Gly Asn Gly Xaa Xaa Lys Cys Phe Gly Pro Gln
   20 25 30
   Cys Leu Cys Asn Arg
   35
<210> 32
   <211> 35
   <212> PRT
   <213> Artificial sequence
<220>
   <223> Insectotoxin 15A consensus sequence
<220>
   <221> MISC_FEATURE
   <222> (10)..(10)
   <223> Xaa can be His or Pro
<220>
   <221> MISC_FEATURE
   <222> (17)..(17)
   <223> Xaa can be Asp or Arg
<220>
   <221> MISC_FEATURE
   <222> (23)..(23)
   <223> Xaa can be Lys or Asn
<220>
   <221> MISC_FEATURE
   <222> (25)..(26)
   <223> Xaa can be any amino acid
<400> 32
   Met Cys Met Pro Cys Phe Thr Thr Asp Xaa Asn Met Ala Lys Lys Cys
   1 5 10 15
   Xaa Asp Cys Cys Gly Gly Xaa Gly Xaa Xaa Lys Cys Phe Gly Pro Gln
   20 25 30
   Cys Leu Cys
   35
<210> 33
   <211> 35
   <212> PRT
   <213> Mesobuthus eupeus
<400> 33
   Met Cys Met Pro Cys Phe Thr Thr Asp Pro Asn Met Ala Lys Lys Cys
   1 5 10 15
   Arg Asp Cys Cys Gly Gly Asn Gly Lys Cys Phe Gly Pro Gln Cys Leu
   20 25 30
   Cys Asn Arg
   35
<210> 34
   <211> 33
   <212> PRT
   <213> Artificial sequence
<220>
   <223> Insectotoxin 15A consensus sequence
<220>
   <221> MISC_FEATURE
   <222> (10)..(10)
   <223> Xaa can be His or Pro
<220>
   <221> MISC_FEATURE
   <222> (17)..(17)
   <223> Xaa can be Asp or Arg
<220>
   <221> MISC_FEATURE
   <222> (23)..(23)
   <223> Xaa can be Lys or Asn
<220>
   <221> MISC_FEATURE
   <222> (26)..(26)
   <223> Xaa can be Gly or Cys
<220>
   <221> MISC_FEATURE
   <222> (27)..(27)
   <223> Xaa can be Lys or Phe
<220>
   <221> MISC_FEATURE
   <222> (28)..(28)
   <223> Xaa can be Cys or Gly
<220>
   <221> MISC_FEATURE
   <222> (29)..(29)
   <223> Xaa can be Tyr or Pro
<220>
   <221> MISC_FEATURE
   <222> (30)..(30)
   <223> Xaa can be Gly or Gln
<220>
   <221> MISC_FEATURE
   <222> (31)..(31)
   <223> Xaa can be Pro or Cys
<220>
   <221> MISC_FEATURE
   <222> (32)..(32)
   <223> Xaa can be Gln or Leu
<400> 34
   Met Cys Met Pro Cys Phe Thr Thr Asp Xaa Asn Met Ala Lys Lys Cys
   1 5 10 15
   Xaa Asp Cys Cys Gly Gly Xaa Gly Lys Xaa Xaa Xaa Xaa Xaa Xaa Xaa
   20 25 30
   Cys
<210> 35
   <211> 37
   <212> PRT
   <213> Androctonus mauretanicus
<220>
   <221> MISC_FEATURE
   <222> (1)..(37)
   <223> Xaa can be any amino acid
<400> 35
   Cys Gly Pro Cys Phe Thr Thr Asp Pro Tyr Thr Glu Ser Lys Cys Ala
   1 5 10 15
   Thr Cys Cys Gly Gly Xaa Xaa Arg Gly Lys Cys Val Gly Pro Gln Cys
   20 25 30
   Leu Cys Asn Arg Ile
   35
<210> 36
   <211> 34
   <212> PRT
   <213> Artificial sequence
<220>
   <223> Neurotoxin P2 consensus sequence
<220>
   <221> MISC_FEATURE
   <222> (2)..(2)
   <223> Xaa can be Met or Gly
<220>
   <221> MISC_FEATURE
   <222> (9)..(9)
   <223> Xaa can be His or Pro
<220>
   <221> MISC_FEATURE
   <222> (10)..(10)
   <223> Xaa can be Gln or Tyr
<220>
   <221> MISC_FEATURE
   <222> (11)..(11)
   <223> Xaa can be Met or Thr
<220>
   <221> MISC_FEATURE
   <222> (12)..(12)
   <223> Xaa can be Ala or Glu
<220>
   <221> MISC_FEATURE
   <222> (13)..(13)
   <223> Xaa can be Arg or Ser
<220>
   <221> MISC_FEATURE
   <222> (16)..(16)
   <223> Xaa can be Asp or Ala
<220>
   <221> MISC_FEATURE
   <222> (17)..(17)
   <223> Xaa can be Asp or Thr
<220>
   <221> MISC_FEATURE
   <222> (22)..(23)
   <223> Xaa can be any amino acid
<220>
   <221> MISC_FEATURE
   <222> (28)..(28)
   <223> Xaa can be Tyr or Val
<400> 36
   Cys Xaa Pro Cys Phe Thr Thr Asp Xaa Xaa Xaa Xaa Xaa Lys Cys Xaa
   1 5 10 15
   Xaa Cys Cys Gly Gly Xaa Xaa Arg Gly Lys Cys Xaa Gly Pro Gln Cys
   20 25 30
   Leu Cys
<210> 37
   <211> 35
   <212> PRT
   <213> Androctonus mauretanicus
<400> 37
   Cys Gly Pro Cys Phe Thr Thr Asp Pro Tyr Thr Glu Ser Lys Cys Ala
   1 5 10 15
   Thr Cys Cys Gly Gly Arg Gly Lys Cys Val Gly Pro Gln Cys Leu Cys
   20 25 30
   Asn Arg Ile
   35
<210> 38
   <211> 32
   <212> PRT
   <213> Artificial sequence
<220>
   <223> Neurotoxin P2 consensus sequence
<220>
   <221> MISC_FEATURE
   <222> (2)..(2)
   <223> Xaa can be Met or Gly
<220>
   <221> MISC_FEATURE
   <222> (9)..(9)
   <223> Xaa can be His or Pro
<220>
   <221> MISC_FEATURE
   <222> (10)..(10)
   <223> Xaa can be Gln or Tyr
<220>
   <221> MISC_FEATURE
   <222> (11)..(11)
   <223> Xaa ca be Met or Thr
<220>
   <221> MISC_FEATURE
   <222> (12)..(12)
   <223> Xaa can be Ala or Glu
<220>
   <221> MISC_FEATURE
   <222> (13)..(13)
   <223> Xaa can be Arg or Ser
<220>
   <221> MISC_FEATURE
   <222> (16)..(16)
   <223> Xaa can be Asp or Ala
<220>
   <221> MISC_FEATURE
   <222> (17)..(17)
   <223> Xaa can be Asp or Thr
<220>
   <221> MISC_FEATURE
   <222> (25)..(25)
   <223> Xaa can be Gly or Cys
<220>
   <221> MISC_FEATURE
   <222> (26)..(26)
   <223> Xaa can be Lys or Val
<220>
   <221> MISC_FEATURE
   <222> (27)..(27)
   <223> Xaa can be Cys or Gly
<220>
   <221> MISC_FEATURE
   <222> (28)..(28)
   <223> Xaa can be Tyr or Pro
<220>
   <221> MISC_FEATURE
   <222> (29)..(29)
   <223> Xaa can be Gly or Gln
<220>
   <221> MISC_FEATURE
   <222> (30)..(30)
   <223> Xaa can be Pro or Cys
<220>
   <221> MISC_FEATURE
   <222> (31)..(31)
   <223> Xaa can be Gln or Leu
<400> 38
   Cys Xaa Pro Cys Phe Thr Thr Asp Xaa Xaa Xaa Xaa Xaa Lys Cys Xaa
   1 5 10 15
   Xaa Cys Cys Gly Gly Lys Gly Lys Xaa Xaa Xaa Xaa Xaa Xaa Xaa Cys
   20 25 30
<210> 39
   <211> 37
   <212> PRT
   <213> Artificial sequence
<220>
   <223> Toxin consensus sequence
<220>
   <221> MISC_FEATURE
   <222> (1)..(37)
   <223> Xaa can be any amino acid
<400> 39
   Met Cys Met Pro Cys Phe Thr Thr Asp Pro Asn Met Ala Lys Lys Cys
   1 5 10 15
   Arg Asp Cys Cys Gly Gly Lys Gly Xaa Xaa Lys Cys Phe Gly Pro Gln
   20 25 30
   Cys Leu Cys Asn Arg
   35
<210> 40
   <211> 35
   <212> PRT
   <213> Artificial sequence
<220>
   <223> Toxin consensus sequence
<220>
   <221> MISC_FEATURE
   <222> (3)..(3)
   <223> Xaa can be Met, Lys or Ser
<220>
   <221> MISC_FEATURE
   <222> (10)..(10)
   <223> Xaa can be His, Pro, or Gln
<220>
   <221> MISC_FEATURE
   <222> (17)..(17)
   <223> Xaa can be Asp, Ala, or Tyr
<400> 40
   Arg Cys Xaa Pro Cys Phe Thr Thr Asp Xaa Gln Met Ser Lys Lys Cys
   1 5 10 15
   Xaa Asp Cys Cys Gly Gly Lys Gly Lys Gly Lys Cys Tyr Gly Pro Gln
   20 25 30
   Cys Leu Cys
   35
<210> 41
   <211> 35
   <212> PRT
   <213> Artificial sequence
<220>
   <223> Toxin consensus sequence
<400> 41
   Met Cys Met Pro Cys Phe Thr Thr Asp Pro Asn Met Ala Arg Lys Cys
   1 5 10 15
   Arg Asp Cys Cys Gly Gly Arg Gly Lys Cys Phe Gly Pro Gln Cys Leu
   20 25 30
   Cys Asn Arg
   35
<210> 42
   <211> 10
   <212> PRT
   <213> Artificial sequence
<220>
   <223> Pep8-Ctlx
<400> 42
   Cys Gly Gly Lys Gly Arg Gly Lys Cys Tyr
   1 5 10
<210> 43
   <211> 10
   <212> PRT
   <213> Artificial sequence
<220>
   <223> Pep8-SCX1_BUTSI
<400> 43
   Cys Gly Gly Lys Gly Lys Gly Lys Cys Tyr
   1 5 10
<210> 44
   <211> 10
   <212> PRT
   <213> Artificial sequence
<220>
   <223> Pep8-AF079059_2
<400> 44
   Cys Gly Gly Ile Gly Lys Cys Phe Gly Pro
   1 5 10
<210> 45
   <211> 12
   <212> PRT
   <213> Artificial sequence
<220>
   <223> Chlorotoxin Peptide 8 consensus sequence
<220>
   <221> MISC_FEATURE
   <222> (4)..(4)
   <223> Xaa can be Lys or Ile
<400> 45
   Cys Gly Gly Xaa Gly Arg Gly Lys Cys Phe Gly Pro
   1 5 10
<210> 46
   <211> 6
   <212> PRT
   <213> Artificial sequence
<220>
   <223> Chlorotoxin Peptide 8 consensus sequence
<220>
   <221> MISC_FEATURE
   <222> (4)..(4)
   <223> Xaa can be Lys or Ile
<400> 46
   Cys Gly Gly Xaa Gly Lys
   1 5
<210> 47
   <211> 10
   <212> PRT
   <213> Artificial sequence
<220>
   <223> Pep8-NJ0361 sequence
<400> 47
   Cys Gly Gly Gly Lys Lys Cys Phe Gly Pro
   1 5 10
<210> 48
   <211> 12
   <212> PRT
   <213> Artificial sequence
<220>
   <223> Chlorotoxin Peptide 8 consensus sequence
<400> 48
   Cys Gly Gly Lys Gly Lys Gly Lys Cys Phe Gly Pro
   1 5 10
<210> 49
   <211> 6
   <212> PRT
   <213> Artificial sequence
<220>
   <223> Chlorotoxin Peptide 8 consensus sequence
<220>
   <221> MISC_FEATURE
   <222> (4)..(5)
   <223> Xaa can be Lys or Gly
<400> 49
   Cys Gly Gly Xaa Xaa Lys
   1 5
<210> 50
   <211> 10
   <212> PRT
   <213> Artificial sequence
<220>
   <223> Pep8-SCX1_BUTEU sequence
<400> 50
   Cys Lys Gly Arg Gly Lys Cys Phe Gly Pro
   1 5 10
<210> 51
   <211> 12
   <212> PRT
   <213> Artificial sequence
<220>
   <223> Chlorotoxin Peptide 8 consensus sequence
<220>
   <221> MISC_FEATURE
   <222> (3)..(3)
   <223> Xaa can be Gly or Cys
<400> 51
   Cys Gly Xaa Lys Gly Arg Gly Lys Cys Phe Gly Pro
   1 5 10
<210> 52
   <211> 6
   <212> PRT
   <213> Artificial sequence
<220>
   <223> Chlorotoxin Peptide 8 consensus sequence
<220>
   <221> MISC_FEATURE
   <222> (2)..(2)
   <223> Xaa can be Gly or Lys
<400> 52
   Cys Xaa Gly Lys Gly Lys
   1 5
<210> 53
   <211> 10
   <212> PRT
   <213> Artificial sequence
<220>
   <223> Pep8-SCX5_BUTEU sequence
<400> 53
   Cys Gly Gly Asn Gly Lys Cys Phe Gly Pro
   1 5 10
<210> 54
   <211> 12
   <212> PRT
   <213> Artificial sequence
<220>
   <223> Chlorotoxin Peptide 8 consensus sequence
<220>
   <221> MISC_FEATURE
   <222> (4)..(4)
   <223> Xaa can be Lys or Asn
<400> 54
   Cys Gly Gly Xaa Gly Arg Gly Lys Cys Phe Gly Pro
   1 5 10
<210> 55
   <211> 6
   <212> PRT
   <213> Artificial sequence
<220>
   <223> Chlorotoxin Peptide 8 consensus sequence
<220>
   <221> MISC_FEATURE
   <222> (4)..(4)
   <223> Xaa can be Lys or Asn
<400> 55
   Cys Gly Gly Xaa Gly Lys
   1 5
<210> 56
   <211> 10
   <212> PRT
   <213> Artificial sequence
<220>
   <223> Pep8-SCXP_ANDMA sequence
<400> 56
   Cys Gly Gly Arg Gly Lys Cys Val Gly Pro
   1 5 10
<210> 57
   <211> 12
   <212> PRT
   <213> Artificial sequence
<220>
   <223> Chlorotoxin Peptide 8 consensus sequence
<220>
   <221> MISC_FEATURE
   <222> (10)..(10)
   <223> Xaa can be Tyr or Val
<400> 57
   Cys Gly Gly Lys Gly Arg Gly Lys Cys Xaa Gly Pro
   1 5 10
<210> 58
   <211> 6
   <212> PRT
   <213> Artificial sequence
<220>
   <223> Chlorotoxin Peptide 8 consensus sequence
<400> 58
   Cys Gly Gly Lys Gly Lys
   1 5
<210> 59
   <211> 12
   <212> PRT
   <213> Artificial sequence
<220>
   <223> Chlorotoxin Peptide 8 consensus sequence
<220>
   <221> MISC_FEATURE
   <222> (4)..(5)
   <223> Xaa can be Lys or Gly
<400> 59
   Cys Gly Gly Xaa Xaa Arg Gly Lys Cys Phe Gly Pro
   1 5 10
<210> 60
   <211> 10
   <212> PRT
   <213> Artificial sequence
<220>
   <223> Chlorotoxin Peptide 8 consensus sequence
<400> 60
   Cys Gly Gly Lys Gly Lys Cys Phe Gly Pro
   1 5 10
<210> 61
   <211> 10
   <212> PRT
   <213> Artificial sequence
<220>
   <223> Chlorotoxin Peptide 21 sequence
<400> 61
   Thr Thr Asp His Gln Met Ala Arg Lys Cys
   1 5 10
<210> 62
   <211> 10
   <212> PRT
   <213> Artificial sequence
<220>
   <223> Pep21-SCX1-BUTSI sequence
<400> 62
   Thr Thr Asp Pro Gln Met Ser Lys Lys Cys
   1 5 10
<210> 63
   <211> 10
   <212> PRT
   <213> Artificial sequence
<220>
   <223> Chlorotoxin Peptide 21 consensus sequence
<220>
   <221> MISC_FEATURE
   <222> (4)..(4)
   <223> Xaa can be His or Pro
<400> 63
   Thr Thr Asp Xaa Gln Met Ala Lys Lys Cys
   1 5 10
<210> 64
   <211> 10
   <212> PRT
   <213> Artificial sequence
<220>
   <223> Pep21-SCX8_LEIQH sequence
<400> 64
   Thr Thr Asp Gln Gln Met Thr Lys Lys Cys
   1 5 10
<210> 65
   <211> 10
   <212> PRT
   <213> Artificial sequence
<220>
   <223> Chlorotoxin Peptide 21 consensus sequence
<220>
   <221> MISC_FEATURE
   <222> (4)..(4)
   <223> Xaa can be His or Gln
<220>
   <221> MISC_FEATURE
   <222> (7)..(7)
   <223> Xaa can be Ala or Thr
<400> 65
   Thr Thr Asp Xaa Gln Met Xaa Lys Lys Cys
   1 5 10
<210> 66
   <211> 10
   <212> PRT
   <213> Artificial sequence
<220>
   <223> Pep21-AF079059_2 sequence
<400> 66
   Thr Thr Asp Ala Asn Met Ala Arg Lys Cys
   1 5 10
<210> 67
   <211> 10
   <212> PRT
   <213> Artificial sequence
<220>
   <223> Chlorotoxin Peptide 21 consensus sequence
<220>
   <221> MISC_FEATURE
   <222> (4)..(4)
   <223> Xaa can be His or Ala
<400> 67
   Thr Thr Asp Xaa Asn Met Ala Arg Lys Cys
   1 5 10
<210> 68
   <211> 10
   <212> PRT
   <213> Artificial sequence
<220>
   <223> Pep21-JN0361 sequence
<400> 68
   Thr Thr Asp Pro Asn Met Ala Asn Lys Cys
   1 5 10
<210> 69
   <211> 10
   <212> PRT
   <213> Artificial sequence
<220>
   <223> Chlorotoxin Peptide 21 consensus sequence
<220>
   <221> MISC_FEATURE
   <222> (4)..(4)
   <223> Xaa can be either His or Pro
<220>
   <221> MISC_FEATURE
   <222> (8)..(8)
   <223> Xaa can be Arg or Asn
<400> 69
   Thr Thr Asp Xaa Asn Met Ala Xaa Lys Cys
   1 5 10
<210> 70
   <211> 10
   <212> PRT
   <213> Artificial sequence
<220>
   <223> Pep21-SCX1_BUTEU sequence
<400> 70
   Thr Thr Arg Pro Asp Met Ala Gln Gln Cys
   1 5 10
<210> 71
   <211> 10
   <212> PRT
   <213> Artificial sequence
<220>
   <223> Chlorotoxin Peptide 21 consensus sequence
<220>
   <221> MISC_FEATURE
   <222> (3)..(3)
   <223> Xaa can be Asp or Arg
<220>
   <221> MISC_FEATURE
   <222> (4)..(4)
   <223> Xaa can be His or Pro
<220>
   <221> MISC_FEATURE
   <222> (5)..(5)
   <223> Xaa can be Gln or Asp
<220>
   <221> MISC_FEATURE
   <222> (8)..(8)
   <223> Xaa can be Arg or Gln
<220>
   <221> MISC_FEATURE
   <222> (9)..(9)
   <223> Xaa can be Lys or Gln
<400> 71
   Thr Thr Xaa Xaa Xaa Met Ala Xaa Xaa Cys
   1 5 10
<210> 72
   <211> 10
   <212> PRT
   <213> Artificial sequence
<220>
   <223> Pep21-SCX5_BUTEU sequence
<400> 72
   Thr Thr Asp Pro Asn Met Ala Lys Lys Cys
   1 5 10
<210> 73
   <211> 10
   <212> PRT
   <213> Artificial sequence
<220>
   <223> Chlorotoxin Peptide 21 consensus sequence
<220>
   <221> MISC_FEATURE
   <222> (4)..(4)
   <223> Xaa can be His or Pro
<400> 73
   Thr Thr Asp Xaa Asn Met Ala Lys Lys Cys
   1 5 10
<210> 74
   <211> 10
   <212> PRT
   <213> Artificial sequence
<220>
   <223> Pep21-SCXP_ANDMA sequence
<400> 74
   Thr Thr Asp Pro Tyr Thr Glu Ser Lys Cys
   1 5 10
<210> 75
   <211> 10
   <212> PRT
   <213> Artificial sequence
<220>
   <223> Chlorotoxin Peptide 21 consensus sequence
<220>
   <221> MISC_FEATURE
   <222> (4)..(4)
   <223> Xaa can be His or Pro
<220>
   <221> MISC_FEATURE
   <222> (5)..(5)
   <223> Xaa can be Gln or Tyr
<220>
   <221> MISC_FEATURE
   <222> (6)..(6)
   <223> Xaa can be Met or Thr
<220>
   <221> MISC_FEATURE
   <222> (7)..(7)
   <223> Xaa can be Ala or Glu
<220>
   <221> MISC_FEATURE
   <222> (8)..(8)
   <223> Xaa can be Arg or Ser
<400> 75
   Thr Thr Asp Xaa Xaa Xaa Xaa Xaa Lys Cys
   1 5 10
<210> 76
   <211> 10
   <212> PRT
   <213> Artificial sequence
<220>
   <223> Chlorotoxin Peptide 21 consensus sequence
<400> 76
   Thr Thr Asp Pro Asn Met Ala Lys Lys Cys
   1 5 10
<210> 77
   <211> 7
   <212> PRT
   <213> Artificial sequence
<220>
   <223> Chlorotoxin derivative STP-1
<400> 77
   Thr Asp Pro Gln Met Ser Arg
   1 5
<210> 78
   <211> 10
   <212> PRT
   <213> Artificial sequence
<220>
   <223> Peptide 8 sequences
<400> 78
   Gly Gly Lys Gly Arg Gly Lys Ser Tyr Gly
   1 5 10
<210> 79
   <211> 9
   <212> PRT
   <213> Artificial sequence
<220>
   <223> Peptide 8a sequence
<400> 79
   Gly Lys Gly Arg Gly Lys Ser Tyr Gly
   1 5
<210> 80
   <211> 8
   <212> PRT
   <213> Artificial sequence
<220>
   <223> Peptide 8b sequence
<400> 80
   Lys Gly Arg Gly Lys Ser Tyr Gly
   1 5
<210> 81
   <211> 7
   <212> PRT
   <213> Artificial sequence
<220>
   <223> Peptide 8c sequence
<400> 81
   Gly Arg Gly Lys Ser Tyr Gly
   1 5
<210> 82
   <211> 10
   <212> PRT
   <213> Artificial sequence
<220>
   <223> Peptide 21 sequence
<400> 82
   Thr Thr Asp His Gln Met Ala Arg Lys Ser
   1 5 10
<210> 83
   <211> 8
   <212> PRT
   <213> Artificial sequence
<220>
   <223> Peptide 21b sequence
<400> 83
   Asp His Gln Met Ala Arg Lys Ser
   1 5
<210> 84
   <211> 7
   <212> PRT
   <213> Artificial sequence
<220>
   <223> Peptide 21c sequence
<400> 84
   His Gln Met Ala Arg Lys Ser
   1 5
<210> 85
   <211> 6
   <212> PRT
   <213> Artificial sequence
<220>
   <223> Peptide 21d sequence <400> 85
<210> 86
   <211> 9
   <212> PRT
   <213> Artificial sequence
<220>
   <223> Peptide 21a-A1 sequence
<400> 86
   Ala Asp His Gln Met Ala Arg Lys Ser
   1 5
<210> 87
   <211> 9
   <212> PRT
   <213> Artificial sequence
<220>
   <223> Peptide 21a-A2 sequence
<400> 87
   Thr Ala His Gln Met Ala Arg Lys Ser
   1 5
<210> 88
   <211> 9
   <212> PRT
   <213> Artificial sequence
<220>
   <223> Peptide 21a-A3 sequence
<400> 88
   Thr Asp Ala Gln Met Ala Arg Lys Ser
   1 5
<210> 89
   <211> 9
   <212> PRT
   <213> Artificial sequence
<220>
   <223> Peptide 21a-A4 sequence
<400> 89
   Thr Asp His Ala Met Ala Arg Lys Ser
   1 5
<210> 90
   <211> 9
   <212> PRT
   <213> Artificial sequence
<220>
   <223> Peptide 21a-A5 sequence
<400> 90
   Thr Asp His Gln Ala Ala Arg Lys Ser
   1 5
<210> 91
   <211> 9
   <212> PRT
   <213> Artificial sequence
<220>
   <223> Peptide 21a-A7 sequence
<400> 91
   Thr Asp His Gln Met Ala Ala Lys Ser
   1 5
<210> 92
   <211> 9
   <212> PRT
   <213> Artificial sequence
<220>
   <223> Peptide 21a-A8 sequence
<400> 92
   Thr Asp His Gln Met Ala Arg Ala Ser
   1 5
<210> 93
   <211> 9
   <212> PRT
   <213> Artificial sequence
<220>
   <223> Peptide 21a-A9 sequence
<400> 93
   Thr Asp His Gln Met Ala Arg Lys Ala
   1 5
<210> 94
   <211> 9
   <212> PRT
   <213> Mesobuthus tamulus sindicus
<220>
   <221> MISC_FEATURE
   <222> (1)..(9)
   <223> GenBank Accesssion No. P15229, small toxin
<400> 94
   Thr Thr Asp Gln Gln Met Ser Lys Lys
   1 5
<210> 95
   <211> 9
   <212> PRT
   <213> Leiurus quinquestriatus hebraeu
<220>
   <221> Misc_feature
   <222> (1)..(9)
   <223> GenBank Accession No. P55966, probable toxin
<400> 95
   Thr Thr Asp Pro Gln Met Ser Lys Lys
   1 5

## Description

### Field Of The Invention

The present invention relates generally to the fields of cell physiology and oncology. More specifically, the present invention relates to a novel method of treating cell proliferative disorders, such as cancers, with doses of chlorotoxin and/or derivatives thereof in combination with a chemotherapeutic agent.

### Background of the Invention

Tumors that originate in brain tissue are known as primary brain tumors as opposed to secondary brain tumors that develop when cancer metastatizes to the brain. Primary brain tumors are classified by the type of tissue in which they begin. The most common brain tumors are gliomas, which begin in the glial (supportive) tissue. Astrocytomas are a type of glioma that arise from small, star-shaped cells called astrocytes. They may grow anywhere in the brain or spinal cord but most often arise in the cerebrum in adults and the brain stem, the cerebrum, and the cerebellum in children. A grade III astrocytoma is sometimes called anaplastic astrocytoma while a grade IV astrocytoma is usually called glioblastoma multiforme. Brain stem gliomas occur in the lowest, stemlike part of the brain. Tumors in this area generally cannot be removed. Most brain stem gliomas are high-grade astrocytomas. Ependymomas are a type of glioma that usually develop in the lining of the ventricles and may also occur in the spinal cord. Although these tumors can develop at any age, they are most common in childhood and adolescence. Oligodendrogliomas arise in the cells that produce myelin, the fatty covering that protects nerves. These rare tumors usually arise in the cerebrum, grow slowly, usually do not spread into surrounding brain tissue and occur most often in middle-aged adults but have been found in people of all ages.

There are other types of brain tumors that do not originate in glial tissue.

Medulloblastomas were once thought to develop from glial cells. However, recent research suggests that these tumors develop from primitive (developing) nerve cells that normally do not remain in the body after birth. For this reason, medulloblastomas are sometimes called primitive neuroectodermal tumors. Most medulloblastomas arise in the cerebellum, however, they may occur in other areas as well. Meningiomas grow from the meninges and are usually benign. Because these tumors grow very slowly, the brain may be able to adjust to their presence and therefore these tumors often grow quite large before they cause symptoms. Schwannomas are benign tumors that begin in Schwann cells, which produce the myelin that protects the acoustic nerve. Acoustic neuromas are a type of schwannoma and occur mainly in adults. Craniopharyngiomas develop in the region of the pituitary gland near the hypothalamus and are usually benign, however, they are sometimes considered malignant because they can press on or damage the hypothalamus and affect vital functions. Germ cell tumors arise from primitive (developing) sex cells or germ cells. The most frequent type of germ cell tumor in the brain is the germinoma. Pineal region tumors occur in or around the pineal gland, a tiny organ near the center of the brain. The tumor can be slow growing (pineocytoma) or fast growing (pineoblastoma). The pineal region is very difficult to reach, and these tumors often cannot be removed.

Primitive neuroectodermal tumors are found both in the central and peripheral nervous systems. Primitive neuroectodermal tumors found only in the peripheral nervous system are referred to as peripheral primitive neuroectodermal tumors. Primitive neuroectodermal tumors manifest preferentially in children and have capacity for developing into a variety of neuronal, astrocytic, ependymal, muscular and melanotic lines. The conceptual basis of grouping these tumors together is based upon sharing common progenitor cells as well as sharing similar neoplastic transformations leading to tumors of similar morphological features and biological behavior. However, there remains controversy in placing all primitive neuroectodermal tumors into the same categories.

Supratentorial primitive neuroectodermal tumors include cerebral medulloblastomas, cerebral neuroblastomas, ependymoblastoma and other primitive neuroectodermal tumors, such as pineoblastomas. Peripheral neuroblastic tumors of the adrenal gland (medulla) and sympathetic nervous system are the most common type of childhood tumor outside of the central nervous system. Primary sites for these primitive neuroectodermal tumors are in the adrenals, abdominal, thoracic, cervical and pelvic sympathetic ganglia but include other primary sites as orbit, kidney, lung, skin, ovary, spermatic cord, and urinary bladder. Specific names of these related tumors are pheochromocytomas, paraganglioma, neuroblastomas, ganglioneuromas, ganglioneuroblastomas, neurofibromas, schwannomas, and malignant peripheral nerve sheath tumors. These all share common origin in the neural crest. Medulloblastomas are members of the primitive neuroectodermal tumors that are described as highly malignant embryonal tumors of the central nervous system found in the cerebellum.

Currently, surgery is the treatment of choice for tumors of the central nervous system. Surgery provides a definite diagnosis, relieves the mass bulkiness of the tumor and extends survival of the patient. The only post-surgery adjuvant treatment which is known to work effectively on central nervous system tumors is radiation, and it can prolong survival. Radiation treatment, however, has many undesirable side effects. It can damage the normal tissue of the patient, including the neuronal tissue. Radiation also can cause severe side effects (*e.g*., nausea, vomiting, hair loss).

The other common post-surgery adjuvant cancer treatment, chemotherapy, is relatively ineffective against neuroectodermal tumors. For example, chemotherapy against neuroectodermal tumors with nitrosourea agents is not curative. Many other cancer treating agents have been studied and tested, but generally they have a minimal effect on extending survival because many agents do not cross the blood-brain barrier. In view of these limited treatment options, the current prognosis for patients diagnosed with neuroectodermal tumors is not favorable. The median survival term for patients diagnosed with malignant astrocytomas having surgery and no adjuvant treatment is about fourteen weeks. Radiation therapy after surgery extends the median to about thirty-six weeks. The current two year survival rate for all forms of treatment is less than ten percent.

Other types of tumors are also difficult to combat by known cancer treatments. Lung cancer kills more Americans annually than the next four most frequently diagnosed neoplasms combined (Greenlee et al. (2001) CA Cancer J. Clin. 51, 15-36). Approximately eighty percent of primary lung tumors are of the non-small cell variety, which includes squamous cell and large cell carcinomas, as well as adenocarcinomas. Single-modality therapy is considered appropriate for most cases of early and late stage non-small cell lung cancer. Early stage tumors are potentially curable with surgery, chemotherapy, or radiotherapy, and late stage patients usually receive chemotherapy or best supportive care. Intermediate stage or locally advanced non-small cell lung cancer, which comprises twenty-five to thirty-five percent of all cases, is more typically treated with multi-modality therapy.

Breast cancer also presents treatment difficulties using known agents. The incidence of breast cancer in the United States has been rising at a rate of about two percent per year since 1980, and the American Cancer Society estimated that 192,000 cases of invasive breast cancer were diagnosed in 2001. Breast cancer is usually treated with surgery, radiotherapy, chemotherapy, hormone therapy or combinations of the various methods. A major reason for the failure of cancer chemotherapy in breast cancer is the development of resistance to the cytotoxic drugs. Combination therapy using drugs with different mechanisms of action is an accepted method of treatment which prevents development of resistance by the treated tumor. Anti-angiogenic agents are particularly useful in combination therapy because they are not likely to cause resistance development since they do not act on the tumor, but on normal host tissue.

Compositions (see U.S. patent 5,905,027) and methods (see U.S. patent 6,028,174) for diagnosing and treating neuroectodermal tumors (*e.g*., gliomas and meningiomas) have been developed based on the ability of chlorotoxin to bind to tumor cells of neuroectodermal origin (Soroceanu et al. (1998) Cancer Res. 58, 4871-4879; Ullrich et al. (1996) Neuroreport 7, 1020-1024; Ullrich et al. (1996) Am. J. Physiol. 270, C1511-C1521). Diagnosis of neuroectodermal tumors is accomplished by identification of labeled chlorotoxin bound to tumor cells while treatment of neuroectodermal tumors is accomplished by targeting tumors with cytotoxic agents linked to chlorotoxin. Chlorotoxin is a thirty-six amino acid protein naturally derived from *leiurus quinquestriatus* scorpion venom (DeBin et al. (1993) Am. J. Physiol. 264, C361-369). The present invention expands this area of therapeutics by providing a method for treating cancer using chlorotoxin, in combination with other, conventional cancer treating agents.

Soroceanu et al, Journal of Neuroscience, 19(14): 5942-5954, 1999 discloses that chlorotoxin and tamoxifen block the glioma specific chloride channel and inhibit glioma invasion in brain slice invasion assay.

### Summary of the Invention

In a first aspect, the present invention provides a combination comprising (i) chlorotoxin or a chlorotoxin derivative and (ii) temozolomide, for use in the treatment of a neuroectodermal or prostate cancer,
wherein chlorotoxin comprises the sequence set forth in SEQ ID NO: 1, and
wherein the chlorotoxin derivative comprises the sequence TTX₁X₂X₃MX₄X₅K (SEQ ID NO: 13), wherein
(i) X₁ is an acidic amino selected from the group consisting of aspartic acid and glutamic acid;
(ii) X₂ is an amino acid selected from the group consisting of alanine, arginine, asparagine, aspartic acid, cysteine, glutamine, glutamic acid, glycine, histidine, isoleucine, leucine, lysine, proline, methionine, phenylalanine, serine, threonine, tryptophan, tyrosine and valine;
(iii) X₃ is an amide amino acid selected from the group consisting of asparagine and glutamine;
(iv) X₄ is an amino acid selected from the group consisting of serine, threonine and alanine; and
(v) X₅ is a basic amino acid selected from the group consisting of histidine, lysine and arginine.

In a second aspect, the present invention provides the use of (i) chlorotoxin or a chlorotoxin derivative and (ii) temozolomide for the manufacture of a medicament for use in the treatment of a neuroectodermal or prostate cancer,
wherein chlorotoxin comprises the sequence set forth in SEQ ID NO: 1, and
wherein the chlorotoxin derivative comprises the sequence TTX₁X₂X₃MX₄X₅K (SEQ ID NO: 13), wherein
(i) X₁ is an acidic amino selected from the group consisting of aspartic acid and glutamic acid;
(ii) X₂ is an amino acid selected from the group consisting of alanine, arginine, asparagine, aspartic acid, cysteine, glutamine, glutamic acid, glycine, histidine, isoleucine, leucine, lysine, proline, methionine, phenylalanine, serine, threonine, tryptophan, tyrosine and valine;
(iii) X₃ is an amide amino acid selected from the group consisting of asparagine and glutamine;
(iv) X₄ is an amino acid selected from the group consisting of serine, threonine and alanine; and
(v) X₅ is a basic amino acid selected from the group consisting of histidine, lysine and arginine.

In some embodiments chlorotoxin or a chlorotoxin derivative is administered prior to administration of the chemotherapeutic agent, while in other embodiments it is administered simultaneously with the chemotherapeutic agent.

The cancer may be selected from neoplasms of the central nervous system (CNS), spinal axis tumors, glioma, meningioma and pituitary adenoma.

### Brief Description of Figures

Figure 1 depicts the effect of temodar in combination with chlorotoxin *in vitro.* D54 glioma cells were incubated with saline alone (control), temodar alone, temodar plus chlorotoxin, or pretreated with chlorotoxin twenty-four hours prior to temodar treatment.
Figure 2 depicts the effect of chlorotoxin on temodar efficacy *in vivo.* Nude mice with established U251 glioma flank tumors were treated with saline alone (control), temodar alone, or temodar plus chlorotoxin.
Figure 3 depicts the effect of chlorotoxin pretreatment on hydroxyurea efficacy *in vivo.* Nude mice with established D54 glioma flank tumors were treated with saline alone (control), hydroxyurea alone, or chlorotoxin plus hydroxyurea.
Figure 4 depicts a cytotoxicity assay in which low concentrations of chlorotoxin are shown to inhibit the growth and proliferation of glioblastoma cells.
Figure 5 depicts the effect of four day incubation and wash-out on the ability of chlorotoxin to inhibit abnormal cell growth.
Figure 6 depicts a cytotoxicity assay in which low concentrations of chlorotoxin are shown to inhibit the growth and proliferation of prostate cancer cells.
Figure 7 depicts an *in vivo* assay of the ability of chlorotoxin to inhibit the growth of glioblastoma tumor cells in athymic nude mice.
Figure 8 depicts an *in vivo* assay of the ability of chlorotoxin to enhance survival of athymic nude mice with intracranial glioblastoma tumors. Cessation of intravenous treatment indicated by arrow.
Figure 9 depicts an *in vivo* assay of the ability of chlorotoxin to inhibit growth of glioblastoma tumors in the flanks of athymic nude mice.
Figure 10 depicts a series of overlapping 10-mer peptides derived from chlorotoxin. Cysteine residues of SEQ ID NO: 1 are replaced in the 10-mers with serine to prevent cross-linking of peptides.
Figure 11 depicts binding of chlorotoxin and 10-mer peptides 1-15.
Figure 12 depicts binding of chlorotoxin and 10-mer peptides 16-27, 1, 5 and 10.
Figure 13 depicts binding of peptide 21, the native core 9-mer, and each alanine-substituted 9-mer peptide to both U251 and PC3 cells.
Figure 14 depicts binding of short scorpion toxins in PC3 human prostate cancer cells.
Figure 15 depicts the effect of peptide 21 on the proliferation of D54MG cells was studied by adding increasing doses of peptide 21 to the cells and then measuring the uptake of ³H-thymidine.

### Detailed Description

This invention relates to combination chemotherapy, involving temozolomide used in combination with chlorotoxin or a derivative thereof. A cancer cell can be killed by first administering to a cancer cell chlorotoxin in combination with temozolomide. The growth of a tumor may be retarded by administering chlorotoxin to tumor simultaneously with temozolomide. A cancer cell can be killed or the growth of a tumor can be retarded by first administering chlorotoxin (or a chlorotoxin derivative) and subsequently administering temozolomide. The prior, or simultaneous administration of chlorotoxin or a derivative thereof may also have the effect of reducing the amount of temozolomide necessary for successful treatment thus reducing the severe side effects associated with chemotherapeutic agents.

### Combination Chemotherapeutic Compositions

This invention includes a combination for the treatment of neuroectodermal or prostate cancer in a mammal, including a human comprising an amount of chlorotoxin or a chlorotoxin derivative, in combination with temozolomide etc.

As used herein, the term "cancer" unless otherwise indicated, refers to diseases that are characterized by uncontrolled, abnormal cell growth and/or proliferation.

In some aspects, the invention includes a population of conjugate molecules, said conjugate molecules comprising at least one chlorotoxin peptide or a derivative thereof and temozolomide, wherein the extent of conjugation of chlorotoxin and temozolomide is such that the effect of temozolomide in a mammal receiving the conjugate may be enhanced when compared to mixtures of temozolomide with chlorotoxin, or temozolomide alone. In another aspect, the invention includes compositions comprising a population of conjugate molecules wherein at least one chlorotoxin peptide or derivative thereof is conjugated to temozolomide and a pharmaceutically acceptable excipient.

As used herein, the term "chlorotoxin" unless otherwise described, refers to the full-length, thirty-six amino acid polypeptide naturally derived from *Leiurus quinquestriatus* scorpion venom (DeBin et al. (1993) Am. J. Physiol. 264, C361-369) which comprises the amino acid sequence of native chlorotoxin as set forth in SEQ ID NO: 1. The term "chlorotoxin" includes polypeptides comprising SEQ ID NO: 1 which have been synthetically or recombinantly produced, such as those disclosed in U.S. Patent 6,319,891.

As used herein, the term "chlorotoxin subunit" or "subunit of chlorotoxin" refers to a peptide comprising less than thirty-six contiguous amino acids of chlorotoxin and which is capable of specifically binding to cancer cells.

As used herein, the term "chlorotoxin derivative" refers to derivatives as defined above.

Chlorotoxin and peptide derivatives thereof can be prepared using standard solid phase (or solution phase) peptide synthesis methods, as is known in the art. In addition, the nucleic acids encoding these peptides may be synthesized using commercially available oligonucleotide synthesis instrumentation and produced recombinantly using standard recombinant production systems. The production using solid phase peptide synthesis is necessitated if non-gene-encoded amino acids are to be included.

Derivatives of chlorotoxin are polypeptides comprising the amino acid sequence TTX₁X₂X₃MX₄X₅K (SEQ ID NO: 13) corresponding to amino acid residues 7-15 of SEQ ID NO: 1, wherein X₁ is an acidic amino acid selected from the group consisting of aspartic acid and glutamic acid; X₂ is an amino acid selected from the group consisting of alanine, arginine, asparagine, aspartic acid, cysteine, glutamine, glutamic acid, glycine, histidine, isoleucine, leucine, lysine, proline, methionine, phenylalanine, serine, threonine, tryptophan, tyrosine and valine; X₃ is an amide amino acid selected from the group consisting of asparagine and glutamine; X₄ is an any amino acid but in a preferred embodiment is selected from the group consisting of serine, threonine and alanine; and X₅ is a basic amino acid selected from the group consisting of histine, lysine and arginine. In one embodiment, X₁ is aspartic acid, X₂ is histidine or proline, X₃ is glutamine, X₄ is alanine and X₅ is arginine or lysine.

Corresponding polypeptide toxins of other scorpion species display similar or related activity to chlorotoxin. For purposes of the specification, "similar or related activity to chlorotoxin" is defined as binding to cells displaying abnormal cell growth, including benign cells exhibiting abnormal growth and malignant cancer cells. Examples of such polypeptide toxins include, but are not limited to, toxins which contain one or more of the binding domains of chlorotoxin set forth in SEQ ID NO: 8 or SEQ ID NO: 13, and any of the consensus sequences set forth in Table 1.

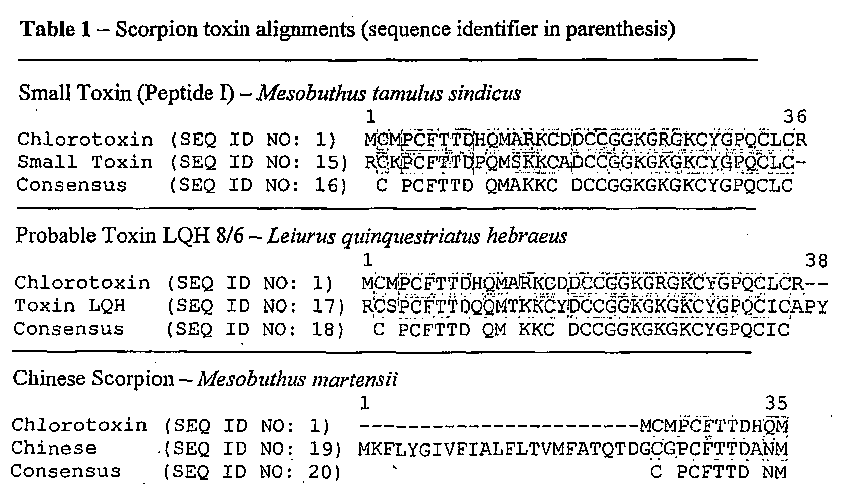

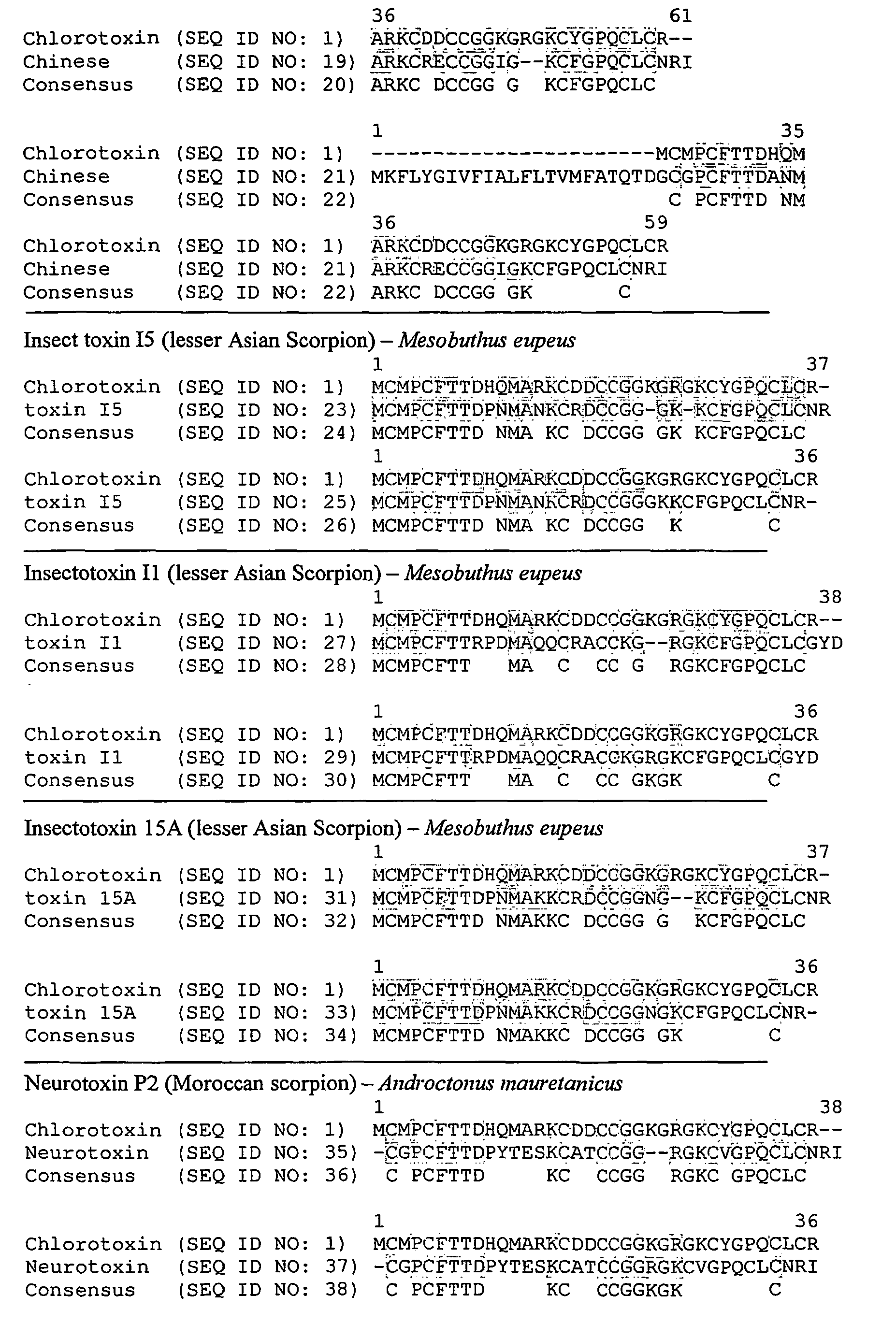

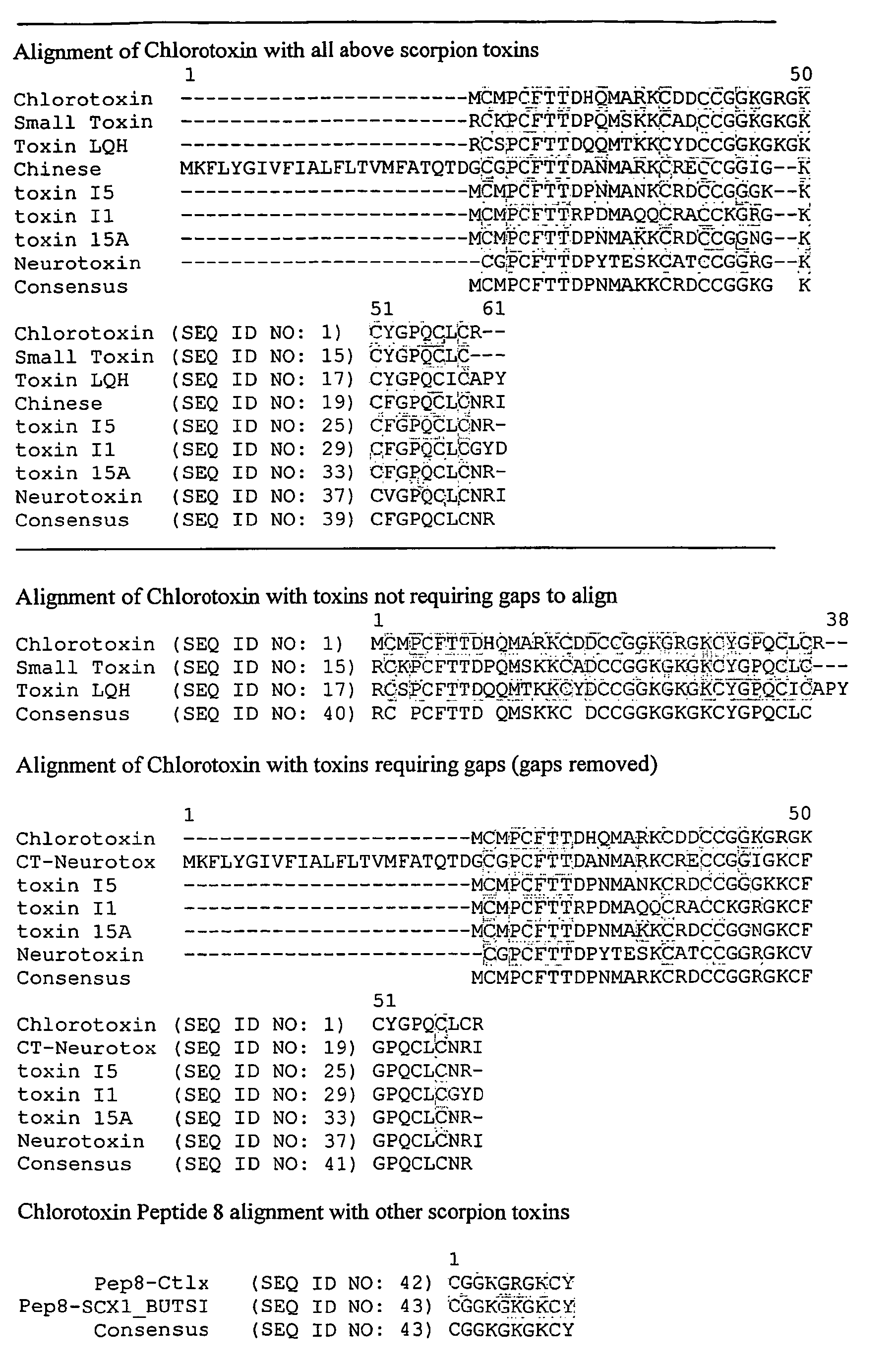

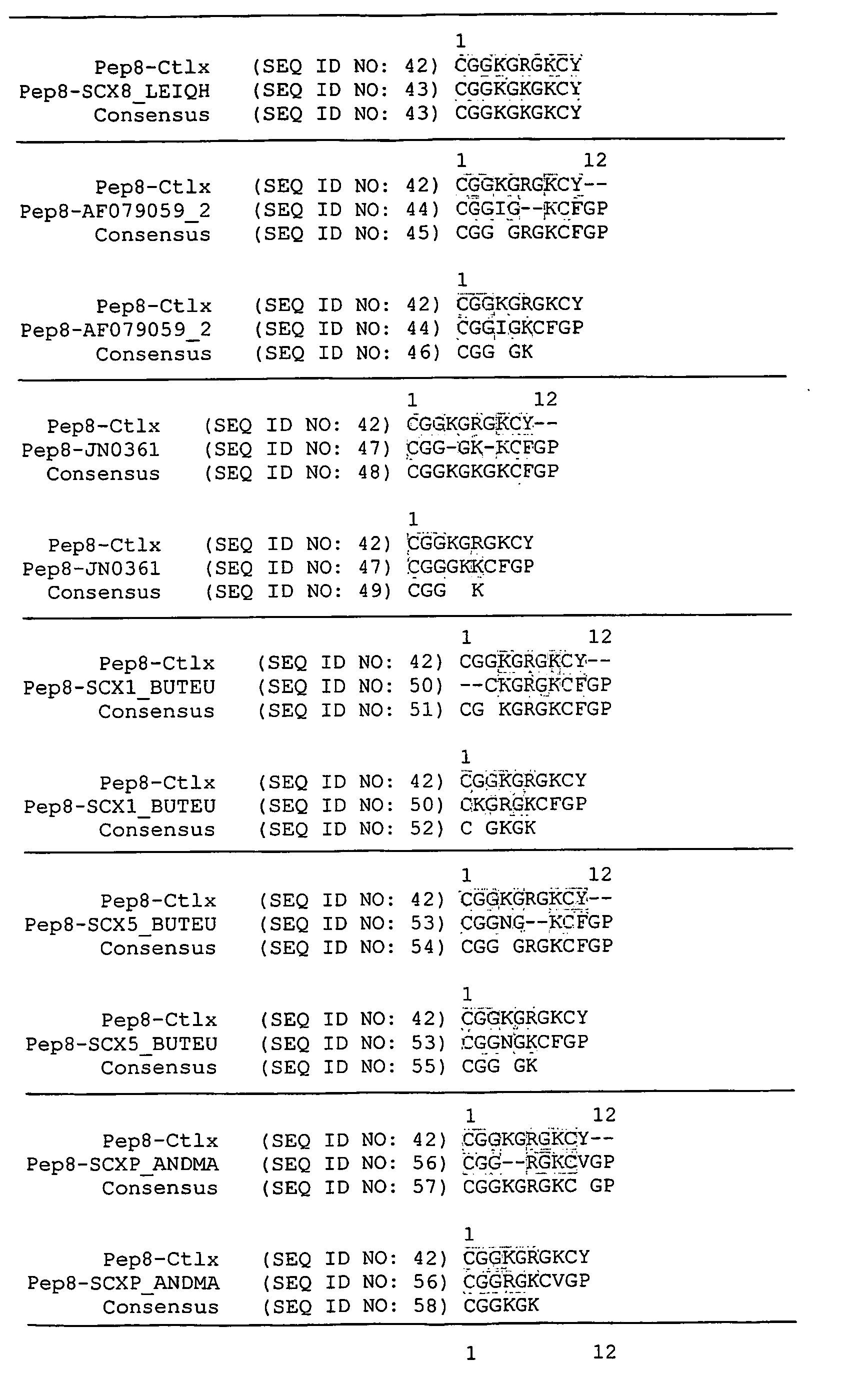

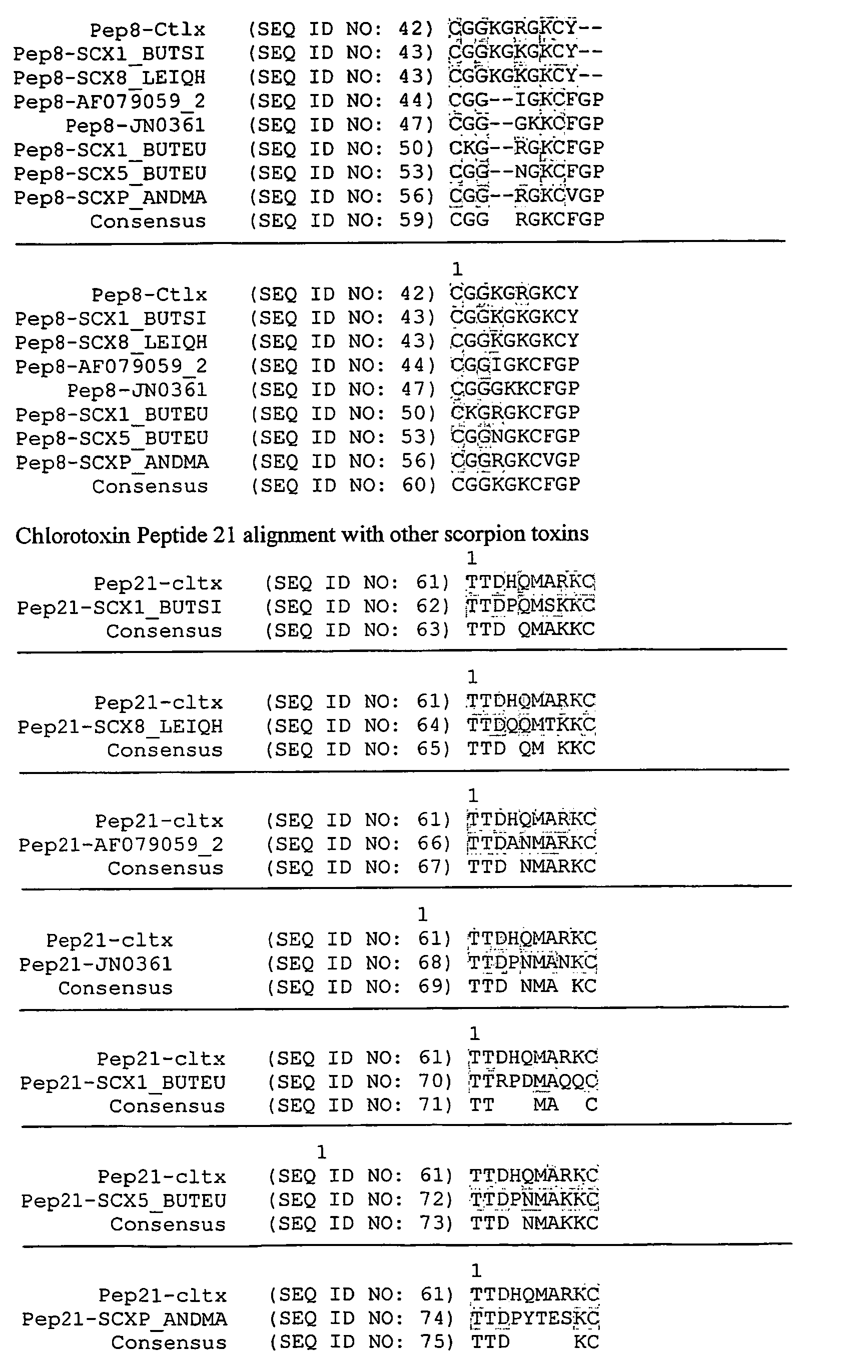

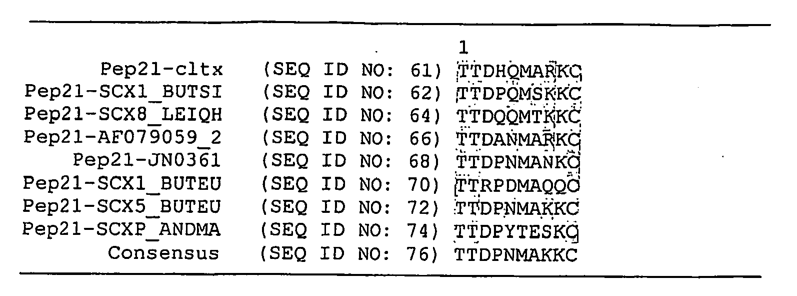

As used herein, the term "related scorpion toxin" refers to any of the toxins or related peptides, such as those disclosed in Table 1, displaying amino acid and/or nucleotide sequence identity to chlorotoxin. Examples of related scorpion toxins include, but are not limited to, CT neurotoxin from *Mesobuthus martensii* (GenBank Accession AAD47373), Neurotoxin BmK 41-2 from *Buthus martensii karsch* (GenBank Accession A59356), Neurotoxin Bm12-b from *Buthus martensii* (GenBank Accession AAK16444), Probable Toxin LQH 8/6 from *Leiurus quinquestriatus hebraeu* (GenBank Accession P55966), Small toxin from *Mesobuthus tamulus sindicus* (GenBank Accession P15229).

Homology or sequence identity at the nucleotide or amino acid sequence level is determined by **BLAST** (Basic Local Alignment Search Tool) analysis using the algorithm employed by the programs **blastp, blastn, blastx, tblastn** and **tblastx** (Altschul et al. (1997) Nucleic Acids Res. 25, 3389-3402 and Karlin et al. (1990) Proc. Natl. Acad. Sci. USA 87, 2264-2268) which are tailored for sequence similarity searching. The approach used by the **BLAST** program is to first consider similar segments, with gaps (non-contiguous) and without gaps (contiguous), between a query sequence and a database sequence, then to evaluate the statistical significance of all matches that are identified and finally to summarize only those matches which satisfy a preselected threshold of significance. For a discussion of basic issues in similarity searching of sequence databases, see Altschul et al. (1994) Nature Genetics 6, 119-129. The search parameters for histogram, descriptions, alignments, expect (i.e., the statistical significance threshold for reporting matches against database sequences), cutoff, matrix and filter (low complexity) are at the default settings. The default scoring matrix used by blastp, blastx, tblastn, and tblastx is the **BLOSUM62** matrix (Henikoff et al. (1992) Proc. Natl. Acad. Sci. USA 89, 10915-10919), recommended for query sequences over eighty-five nucleotides or amino acids in length.

For **blastn**, the scoring matrix is set by the ratios of **M** (*i.e.,* the reward score for a pair of matching residues) to **N** (*i.e.,* the penalty score for mismatching residues), wherein the default values for **M** and **N** are +5 and -4, respectively. Four **blastn** parameters were adjusted as follows: Q=10 (gap creation penalty); R=10 (gap extension penalty); wink=1 (generates word hits at every wink^{th} position along the query); and gapw=16 (sets the window width within which gapped alignments are generated). The equivalent **Blastp** parameter settings were Q=9; R=2; wink= 1; and gapw=32. A **Bestfit** comparison between sequences, available in the GCG package version 10.0, uses DNA parameters GAP=50 (gap creation penalty) and LEN=3 (gap extension penalty) and the equivalent settings in protein comparisons are GAP=8 and LEN=2.

Disclosed herein are allelic variants, conservative substitution variants, and members of the scorpion toxin peptide family, having an amino acid sequence of at least about seventy-five percent, at least about eighty-five percent, at least about ninety percent sequence, at least about ninety-five percent, or at least about ninety-nine percent sequence identity with the entire chlorotoxin sequence set forth in SEQ ID NO: 1. Identity or homology with respect to such sequences is defined herein as the percentage of amino acid residues in the candidate sequence that are identical with the known peptides, after alignment the sequences.

Fusion proteins, or N-terminal or C-terminal extensions, into the peptide sequence shall not be construed as affecting homology. Examples of such extensions include, but are not limited to, the following sequences:
HHHHHHMCMPCFTTDHQMARKCDDCCGGKGRGKCYGPQCLCR SEQ ID NO: 2),
YMCMPCFTTDHQMARKCDDCCGGKGRGKCYGPQCLCR (SEQ ID NO: 3),
YSYMCMPCFTTDHQMARKCDDCCGGKGRGKCYGPQCLCR (SEQ ID NO: 4).
Contemplated peptide variants further, but are not limited to the following sequences:
MCMPCFTTDHQMARKCDDCCGGKGRGKCFGPQCLCR (SEQ ID NO: 5),
RCKPCFTTDPQMSKKCADCCGGKGKGKCYGPQCLC (SEQ ID NO: 6),
RCSPCFTTDQQMTKKCYDCCGGKGKGKCYGPQCICAPY (SEQ ID NO: 7).

### Pharmaceutical Compositions

Pharmaceutical compositions of the present invention can be administered via parenteral, subcutaneous, intravenous, intramuscular, intraperitoneal, transdermal or buccal routes. For example, an agent may be administered locally to a tumor via microinfusion. Alternatively, or concurrently, administration may be by the oral route. For example, chlorotoxin or a derivative thereof could be administered locally to the site of a tumor, followed by oral administration of at least one chemotherapeutic agent. The prior administration of chlorotoxin may have the effect of reducing the amount of chemotherapeutic agent necessary for successful treatment thus reducing the severe side effects associated with chemotherapeutic agents. The dosage administered will be dependent upon the age, health, and weight of the recipient, kind of concurrent treatment, if any, frequency of treatment, and the nature of the effect desired.

While individual needs vary, determination of optimal ranges of effective amounts of each component is within the skill of the art. Typical dosages comprise 1.0 pg/kg body weight to 100 mg/kg body weight. The preferred dosages for systemic administration comprise 100.0 ng/kg body weight to 10.0 mg/kg body weight. The preferred dosages for direct administration to a site via microinfusion comprise 1 ng/kg body weight to 1 mg/kg body weight.

In addition to chlorotoxin and temozolomide, the compositions of the present invention may contain suitable pharmaceutically acceptable carriers comprising excipients and auxiliaries that facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil or synthetic fatty acid esters, for example, ethyl oleate or triglycerides. Aqueous injection suspensions may contain substances which increase the viscosity of the suspension include, for example, sodium carboxymethyl cellulose, sorbitol and dextran. Optionally, the suspension may also contain stabilizers. Liposomes can also be used to encapsulate the agent for delivery into the cell.

The pharmaceutical formulation for systemic administration according to the invention may be formulated for enteral, parenteral or topical administration. Indeed, all three types of formulations may be used simultaneously to achieve systemic administration of the active ingredient.

As mentioned above, topical administration may be used. Any common topical formulation such as a solution, suspension, gel, ointment or salve and the like may be employed. Preparation of such topical formulations are described in the art of pharmaceutical formulations as exemplified, for example, by Gennaro et al. (1995) Remington's Pharmaceutical Sciences, Mack Publishing. For topical application, the compositions could also be administered as a powder or spray, particularly in aerosol form. In a some embodiments, the compositions of this invention may be administered by inhalation. For inhalation therapy the active ingredients may be in a solution useful for administration by metered dose inhalers or in a form suitable for a dry powder inhaler. In another embodiment, the compositions are suitable for administration by bronchial lavage.

Suitable formulations for oral administration include hard or soft gelatin capsules, pills, tablets, including coated tablets, elixirs, suspensions, syrups or inhalations and controlled release forms thereof. In another embodiment, the pharmaceutical composition comprises chlorotoxin or derivatives thereof in combination with at least one sustained release form of chemotherapeutic agent. In such formulations, chlorotoxin or derivatives thereof will be distributed throughout the body, prior to release of the chemotherapeutic agents, allowing for binding of chlorotoxin to the cancer cells prior to binding of the chemotherapeutic agent to the cancer cells. Upon the delayed release of the chemotherapeutic agent from such formulations, and subsequent distribution to the site of the cancer cells, the effects of the chemotherapeutic agent may be enhanced by the earlier binding of chlorotoxin to the cancer cells. Such delayed release formulations may have the same effect as sequential administration of chlorotoxin followed by one or more chemotherapeutic agents.

The combination of the invention may be co-administered along with other chemotherapeutic agents typically prescribed for various types of cancer according to generally accepted oncology medical practice. The compositions of this invention can be utilized *in vivo*, ordinarily in mammals, such as humans, sheep, horses, cattle, pigs, dogs, cats, rats and mice or *in vitro.* The invention is particularly useful in the treatment of human subjects.

Without further description, it is believed that one of ordinary skill in the art can, using the preceding description and the following illustrative examples, make and utilize the compounds of the present invention. The following working examples and are not to be construed as limiting in any way the remainder of the disclosure.

### Example 1

### Determination of chemotherapeutic agent activity in vitro

A tissue culture method was optimized to test the effects of various chemotherapeutic agents on multiple cancer cell lines (see Table 1). Cells were plated on 96-well microtiter tissue culture plates at a density of approximately 1000-2000 cells per well, depending on the specific cell line. Cells were allowed to adhere twenty-four hours in a 37°C, humidified cell culture incubator supplied with five percent carbon dioxide. In order to achieve a dose-response curve for each drug in each cell line, cells were treated with decreasing concentrations of a specific cytotoxic compound for two to five days. Following treatment, the cytotoxic effect of the drug was quantified using the Cell Counting Kit-8 (CCK-8) (Dojindo Inc.) according to the manufacturer's instructions. In brief, following the treatment period with the cytotoxic drug, cells were incubated with CCK-8 reagent and incubated at 37°C for one to four hours, depending on the specific cell type. After incubation, plates were read on a microplate reader at a wavelength of 490 nM. The IC₅₀ of each drug was calculated from a X-Y scatter plot of the negative log concentration of drug versus mean optical density (Table 2).

| **Table 2. Cell Line Designation, Source and Tissue Origin** | | |
|---|---|---|
| **Cell Designation** | **Cell Line Source** | **Tissue Origin** |
| D54-MG | | Human glioblastoma multiforme |
| U251-MG | | Human glioblastoma multiforme |
| SKMEL28 | ATCC (HTB-72) | Human malignant melanoma |
| SKMEL31 | ATCC (HTB-73) | Human malignant melanoma |
| PC-3 | ATCC (CRL-1435) | Human prostate tumor |
| LNCaP | ATCC (CRL-1740) | Human prostate tumor |
| NCI-H187 | ATCC (CRL-5 804) | Human small cell lung carcinoma |

| | | |
|---|---|---|
| **Table 3. IC₅₀ of Chemotherapeutic Agents in Multiple Cell Lines** | | |

| **Cell Line** | **Drug** | **IC₅₀** |
|---|---|---|
| D54-MG | Doxorubicin | 60.0 ng/ml |
| D54-MG | Paclitaxel | 10.5 nM |
| D54-MG | Temodar | 0.12 mM |
| D54-MG | Cisplatin | 0.010 mg/ml |
| D54-MG | 5-Fluorouracil | 0.0015 mg/ml |
| U251 | Doxorubicin | 30.0 ng/ml |
| U251 | Paclitaxel | 8.0 nM |
| U251 | Temodar | 0.15 mM |
| SKMEL28 | Doxorubicin | 40.0 ng/ml |

| **Table 3. IC₅₀ of Chemotherapeutic Agents in Multiple Cell Lines** | | |
|---|---|---|
| SKMEL28 | Temodar | 0.03 mM |
| SKMEL28 | Cisplatin | 0.008 mg/ml |
| SKMEL31 | Doxorubicin | 35.0 ng/ml |
| SKMEL31 | Paclitaxel | 25 nM |
| SKMEL31 | Temodar | 0.15 mM |
| PC-3 | Hydroxyurea | 35 mM |

### Example 2

### Effect of chlorotoxin on chemotherapeutic agent activity in vitro

For measurement of pharmacologic effect of chlorotoxin on chemotherapeutic agents, the cell culture methodology in Example 1 was employed with the following modifications: a concentration of the chemotherapeutic agent approaching the IC₅₀ but usually just below was used in each assay. Various amounts of chlorotoxin were then titrated in combination with a concentration of chemotherapeutic agent near or below its IC₅₀ and the effect of chlorotoxin on the cytotoxic effects of the chemotherapeutic agent measured two to three days following adminstration. Concentration of chlorotoxin employed in this assay ranged from micoromolar down to nanomolar concentrations.

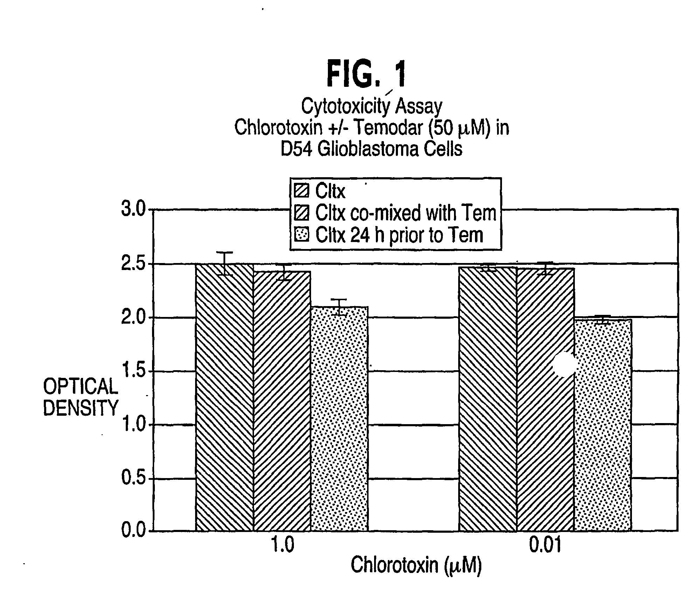

The effect of adding chlorotoxin in combination with Temodar on D54-MG cell proliferation is shown in Figure 1. The level of Temodar used in this experiment (0.050 mM) is about thirty-fold lower than the concentration necessary to kill these cells and produce a lower optical density value (see Table 2). Chloroxotoxin (TM-601) alone had no effect on cell growth. Chlorotoxin when added at the same time as Temodar did not produce any effect but when chlorotoxin was added twenty-four hours prior to Temodar, a concentration of 0.050 mM Temodar reduced cell proliferation equivalent to a level usually observed with a thirty-fold higher concentration of Temodar. These results demonstrate that administration of chlorotoxin, prior to administration of Temodar, sensitized cancer cells to the effects of Temodar.

### Example 3

### Effect of chlorotoxin on chemotherapeutic agent activity in vivo

The purpose of this study was to determine whether hydroxyurea or temodar combined with chlorotoxin were sufficient to inhibit tumor growth as indicated from *in vitro* studies with glioma cell lines. Other studies indicated that chlorotoxin, pre-incubated with human cancer cell lines, greatly sensitized the cells to temodar, a chemotherapeutic, tumor cell killing agent. Combination treatment with chlorotoxin with hydroxyurea or temodar in mice with glioma flank tumors was compared to the treatment group of hydroxyurea or temodar alone and saline alone. Hydroxyurea and temodar dosage was based on the lowest dosage (10 mg/kg body weight) used in previous studies to determine clearance from the body in the treatment of sickle cell disease paradigm in nude mice (Iyamu et al. (2001) Chemotherapy 47, 270-278).

Nude mice were ear-tagged and given an identification number were inoculated with five million U251 glioma cells in 0.10 ml mixture with five percent methyl cellulose under light anesthesia according to standard operating procedures for flank tumor inoculations (Iyamu et al. (2001) Chemotherapy 47, 270-278). Flank tumors had developed and were established approximately thirty days following inoculation.

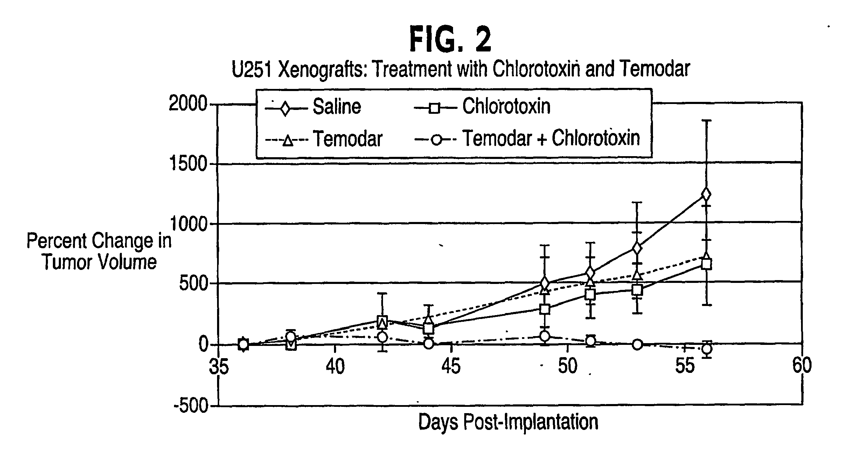

Mice with established flank tumors were each treated with 0.100 ml injection (i.p) of sterilized solutions consisting of either of saline, saline and hydroxyurea or temodar (13.2 mg/kg body weight), or saline, hydroxyurea or temodar (13.2 mg/kg) and chlorotoxin (0.080 mg/kg body weight). Tumor volume was calculated based on the measurements with the same set of calipers on the indicated days by determining the length × width × height of the tumor of non-anesthetized mice. As each animal had different-sized tumors at the beginning of the experiment, the data is presented in final form as percent change of the tumor growth from the initial date of the injection protocol. Statistical significance was determined according to a one-way ANOVA test. At a level where temodar alone has little effect on the growth of the xenografted tumor, temodar combined with chlorotoxin dramatically decreased the growth of the tumor (Figure 2).

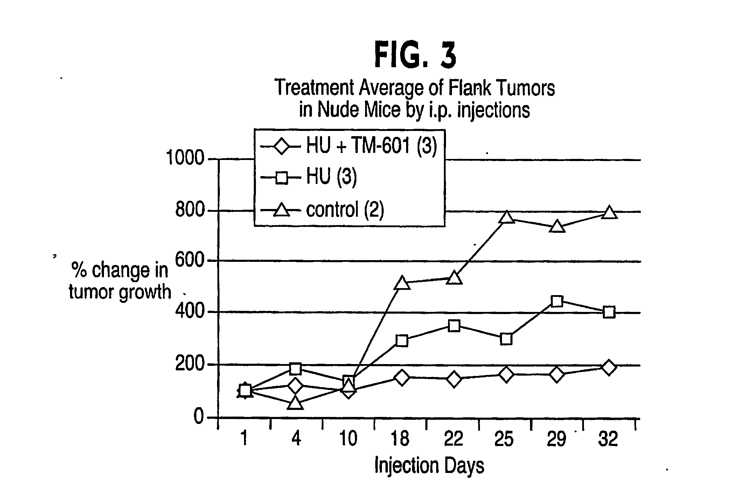

As mentioned above, the efficacy of hyrdroxyurea combined with chlorotoxin was also assessed in the same mouse flank tumor model with the exception that D54 glioma cells were used to establish the glioma flank tumor. Mice treated with chlorotoxin in combination with hydroxyurea had tumors significantly (p=0.01 at day 29 and p=0.005 at day 32) smaller in size than mice treated with either hydroxyurea alone or saline alone indicating that chlorotoxin in combination with hydroxyurea reduced tumor growth significantly more than hydroxyurea alone (Figure 3).

### Example 4

### PET imaging studies with labled chlorotoxin

The following illustrative procedure may be utilized when performing PET imaging studies on patients in the clinic. The patient is fasted for at least twelve hours allowing water intake *ad libitum* and is premedicated with 0.3-0.4 ml Acepromazine injected intra-muscular on the day of the experiment. A twenty-gauge, two inch intravenous catheter is inserted into the contra-lateral ulnar vein for administration of radiolabeled chlorotoxin.

The patient is positioned in the PET camera and a tracer dose of [¹⁵O]H₂O is administered via the intravenous catheter. The image thus obtained is used to insure that the patient is positioned correctly to include complete imaging of the desired areas including the tumor. Subsequently, [⁶⁴Cu] radiolabeled chlorotoxin (<20 mCi) is administered via the intravenous catheter. Following the acquisition of the total radiotracer image, an infusion is begun of the radiolabeled chlorotoxin which is evaluated at multiple dose rates (0.1, 1.0 or 10 mpk/day). After infusion for two hours, the [⁶⁴Cu] radiolabeled chlorotoxin is again injected via the catheter. Images are again acquired for up to ninety minutes. Within ten minutes of the injection of radiotracer and at the end of the imaging session, 1.0 ml blood samples are obtained for determining the plasma concentration of the radiolabeled chlorotoxin.

### Example 5

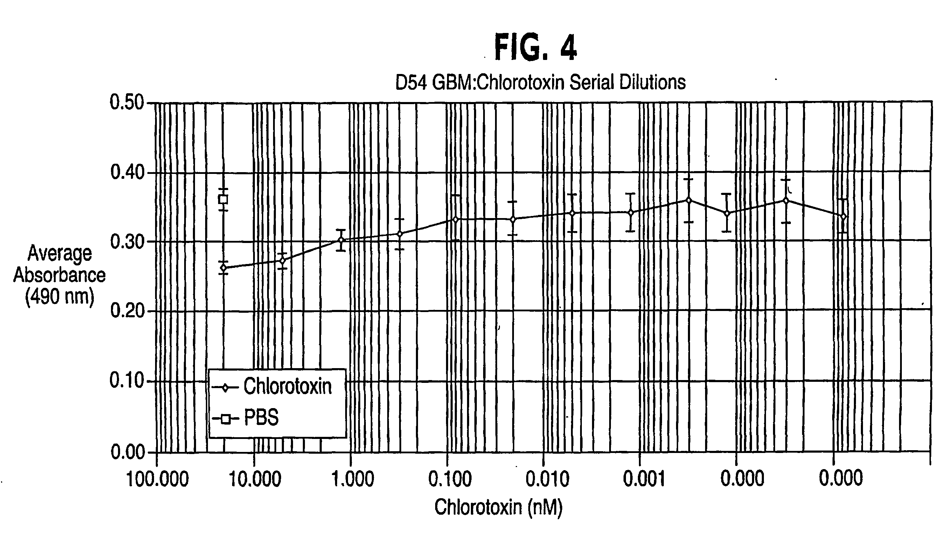

D54 glioblastoma cells were plated at a density of about 1000 cells/well in a 96-well flat bottom plate and incubated in 5% CO₂ at 37°C. After twenty-four hours chlorotoxin was added at 1:4 limiting dilutions to a final concentration of 20, 5, 1.25, 0.313, 0.078, 0.0195, 0.0049, 0.0012, 0.00031 or 0.00008 nM. Control cells received vehicle only. Twenty-four hours after treatment, the effect of chlorotoxin was quantified using the MTT mitochondrial enzyme substrate with the Cell Counting Kit-8 (CCK-8) (Dojindo Inc.) according to the manufacturer's instructions. In brief, following the treatment period with chlorotoxin, cells were incubated with CCK-8 reagent. After incubation, plates were read on a microplate reader at a wavelength of 490 nm, with higher absorbance indicating greater cell viability. Figure 4 shows that chlorotoxin incubation inhibited proliferation of the D54 cells at all concentrations tested down through 0.00120 nM as evidenced by the lower number of viable cells/well versus PBS control.

### Example 6

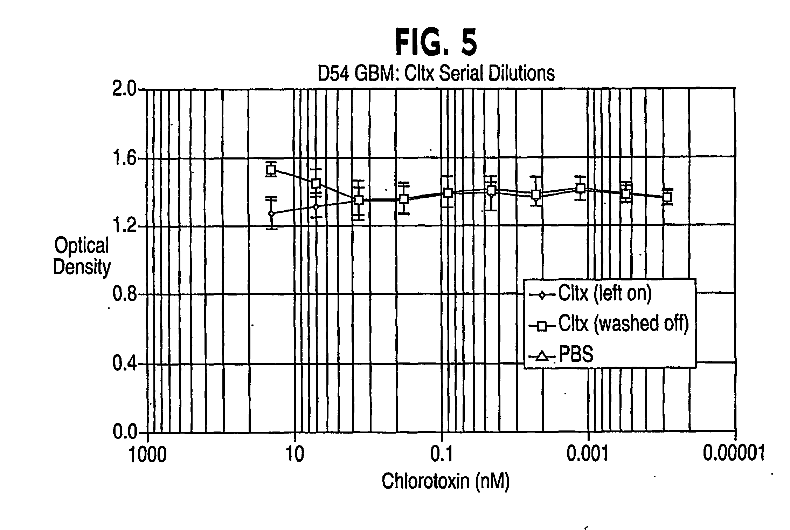

D54 glioblastoma cells were plated at a density of about 1000 cells/well in a 96-well flat bottom plate and incubated in 5% CO₂ at 37°C. After twenty-four hours chlorotoxin was added at 1:4 limiting dilutions to a final concentration (in nM) of 20, 5, 1.25, 0.313, 0.078, 0.02, 0.0049, 0.0012, 0.0003, or 0.00008. Control cells received vehicle only. After twenty-four hours, half of the cells were washed free of chlorotoxin, the medium replaced with fresh medium. Cells in both conditions, chlorotoxin left on and chlorotoxin removed, were incubated for an additional four days. Following incubation, the effect of chlorotoxin was quantified using the MTT mitochondrial enzyme substrate with the CCK-8 as in Example 1. Figure 5 shows that the long incubation time allowed the cells to overcome the effects of chlorotoxin with the additional days of proliferation and chlorotoxin did not appear to inhibit cell proliferation in this instance.

### Example 7

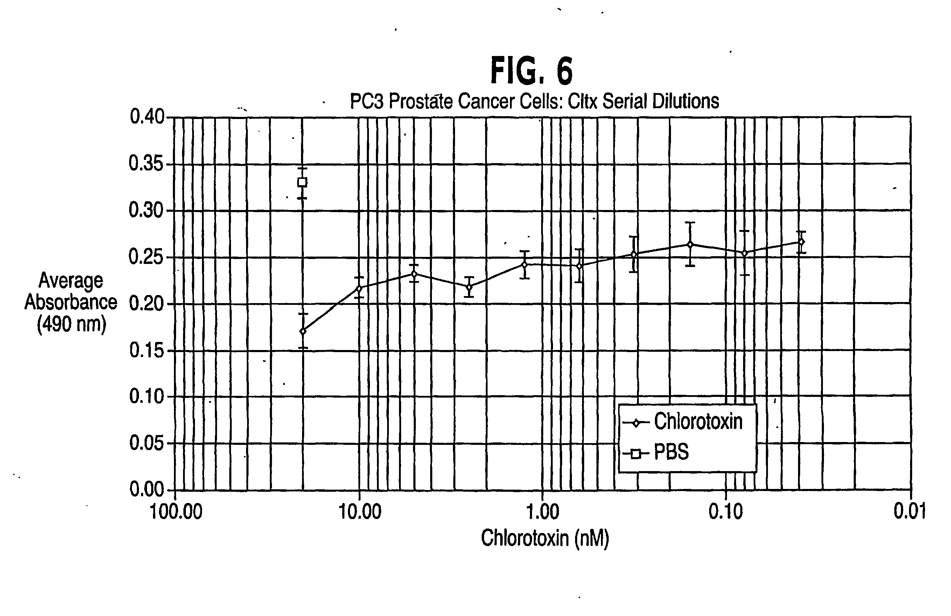

PC3 prostate cancer cells were plated at a density of about 1000 cells/well in a 96-well flat bottom plate and incubated in 5% CO₂ at 37°C. After twenty-four hours chlorotoxin was added at 1:2 limiting dilutions to a final concentration (nM) of 20, 10, 5, 2.5, 1.25, 0.625, 0.313, 0.156,0.078, and 0.039. Control cells received PBS vehicle only. Twenty-four hours after treatment, the effect of chlorotoxin was quantified using the MTT mitochondrial enzyme substrate with the CCK-8 as in Example 1. Figure 6 shows that chlorotoxin incubation inhibited proliferation of the D54 cells at all concentrations tested as evidenced by the lower number of viable cells/well versus PBS control

### Example 8

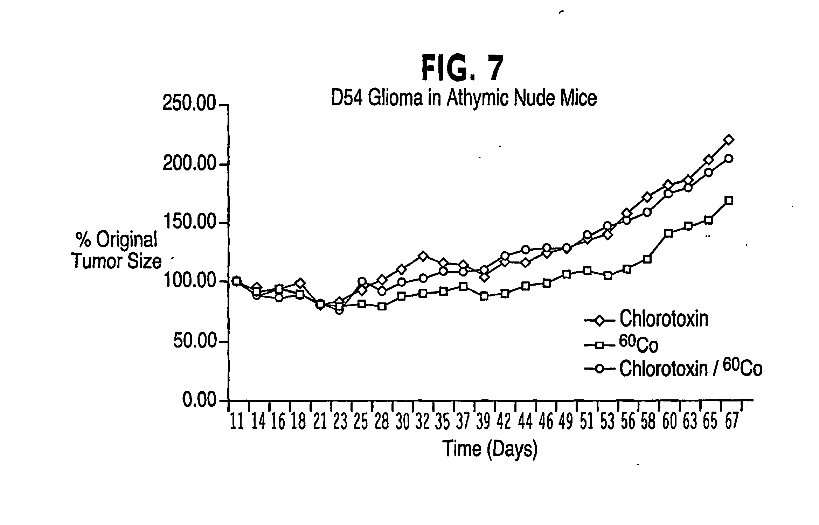

Three groups of eight athymic nude mice received a subcutaneous injection of 5 × 10⁷ human D54 glioblastoma cells in their right flank to produce human glioma flank xenografts in these mice. Animals in Groups I and III received 2.6 µg chlorotoxin (SEQ ID NO: 1) in 100 µl phosphate-buffered saline intravenously at 14, 21, 28, 35, 42 and 49 days after D54 injection. Animals in Groups II and III received 2 Gy ⁶⁰C whole-body irradiation at 15, 22, 29, 36, 43 and 50 days after D54 injection. Tumor size was measured three times weekly and is depicted in Figure 7.

### Example 9

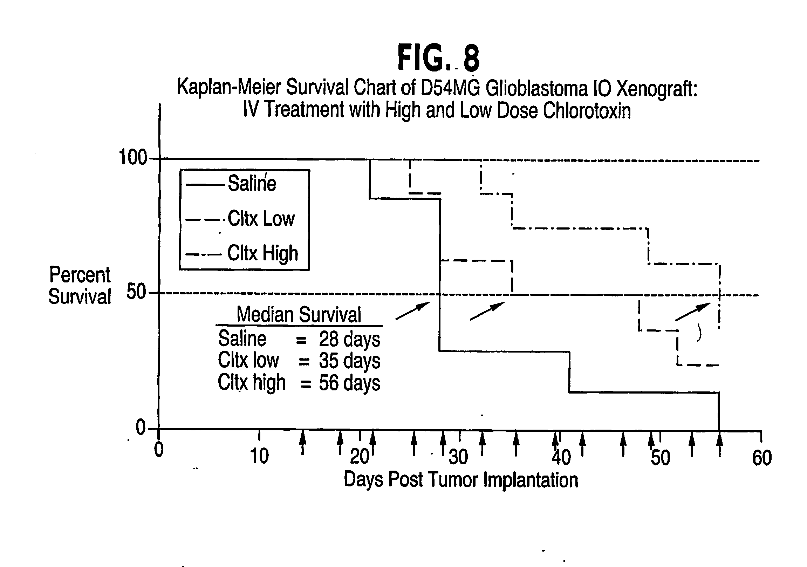

Intracranial D54MG glioma xenografts were established in athymic nude mice by the implantation of 1 × 10⁶ D54MG cells in the brain of each subject. A treatment regimen was begun 14 days post-implantation with tail vein intravenous injections two times per week. The control group of seven mice were administered saline vehicle only. A second group of mice, comprising eight animals, were each administered a low dose of chlorotoxin of 0.2 µg/dose and a third group of mice, comprising eight animals, were administered a high dose of chlorotoxin of 2.0 µg/dose. Animals were followed until death and survival time was plotted on a Kaplan-Meier chart, indicating median survival (Figure 8). These results indicate that treatment with chlorotoxin alone substantially extends the life of a subject in an intracranial model and that this enhanced survival may be dose dependent. It is notable that administration of chlorotoxin was intravenous, demonstrating that chlorotoxin crosses the blood-brain barrier to exert its effect.

### Example 10

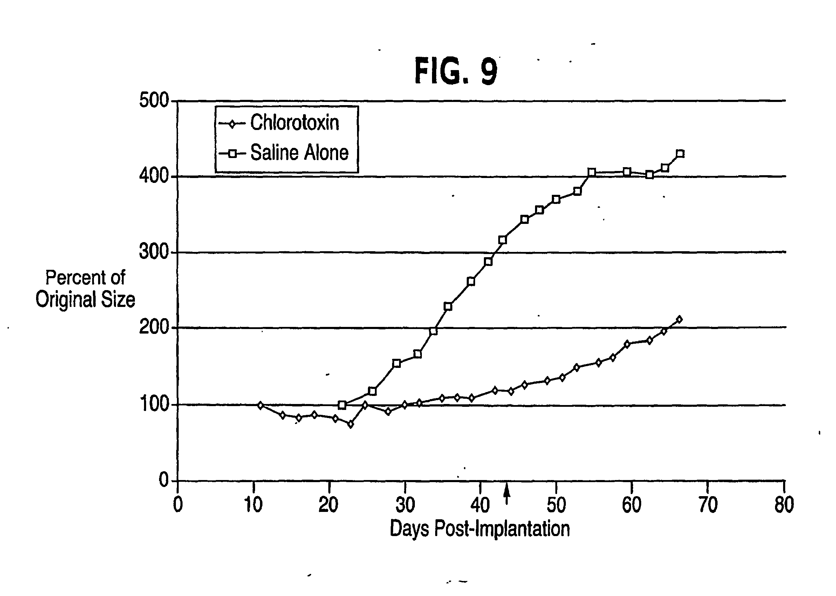

In a separate investigation, D54MG glioma xenografts were established peripherally by implanting 10 × 10⁶ D54MG cells in the flanks of athymic nude mice. Tumors were palpable at 14 days, with individual tumor volumes of approximately 43 mm³. Again, the treatment regimen was begun 14 days post-implantation with tail vein intravenous injections two times per week. The control group of seven mice were administered saline vehicle only. A second group of mice, comprising eight animals, were each administered a low dose of chlorotoxin of 0.2 µg/dose. Tumor size was measured at the time of each injection, and plotted as a percent of original tumor size (Figure 9). Intravenous treatment was ended at 42 days and the measurement of the tumors was continued for several weeks. These results demonstrate that low-dose chlorotoxin alone can dramatically decrease the tumor growth in this flank model.

### Example 11

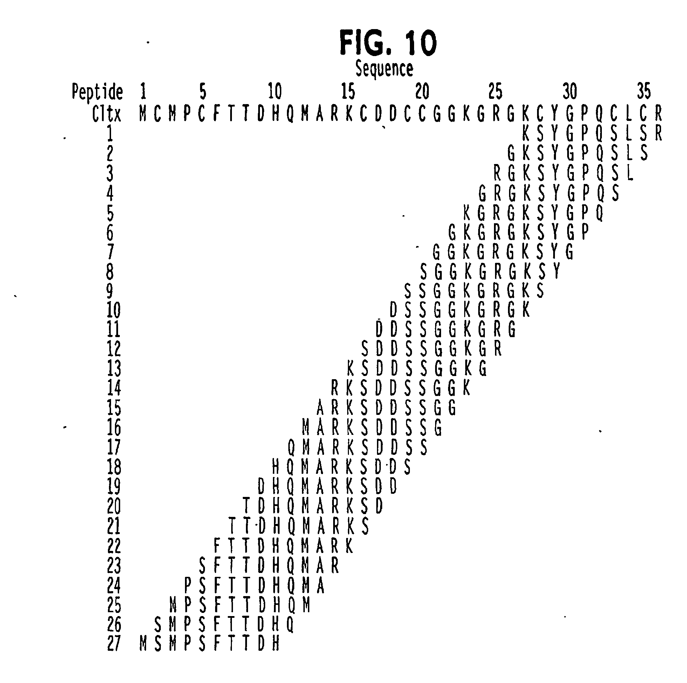

To identify core binding site sequences of chlorotoxin, twenty-seven overlapping 10-mers derived from SEQ ID NO: 1 were synthesized, starting from the C-terminus of the peptide as indicated in Figure 10. Each peptide had a biotin attached at the amino terminus to facilitate detection and each cysteine residue was replaced with a serine in order to prevent cross-linking.

Binding of the 10-mer peptides to PC3 prostate cancer cells *in vitro* was measured by incubating cultured PC3 cells with individual peptides. Binding was detected and quantified by incubating the peptide exposed cells with HRP-avidin using a commercial kit according to manufacturer's instructions.

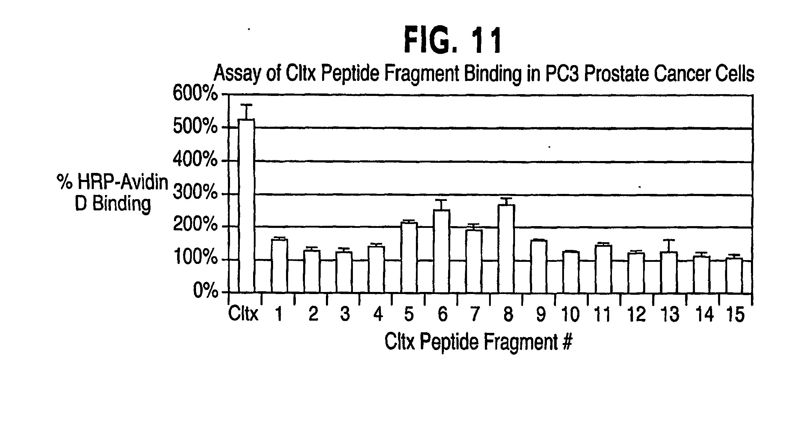

Figure 11 shows that the 10-mer peptide 4 of SEQ ID NO: 1 does not bind to PC3, indicating that the lysine residue which starts peptide 5 must be the start of the binding site. Peptides 5-8 bind, but the binding is lost in peptide 9. This suggests that the tyrosine residue is another key, since this is present in peptide 8 but lost in peptide 9. This indicated that a first binding region of chlorotoxin resides within the 7-mer sequence KGRGKSY (SEQ ID NO: 8) residing at amino acid residues 23-29 of SEQ ID NO: 1 which are common to peptides 5-8.

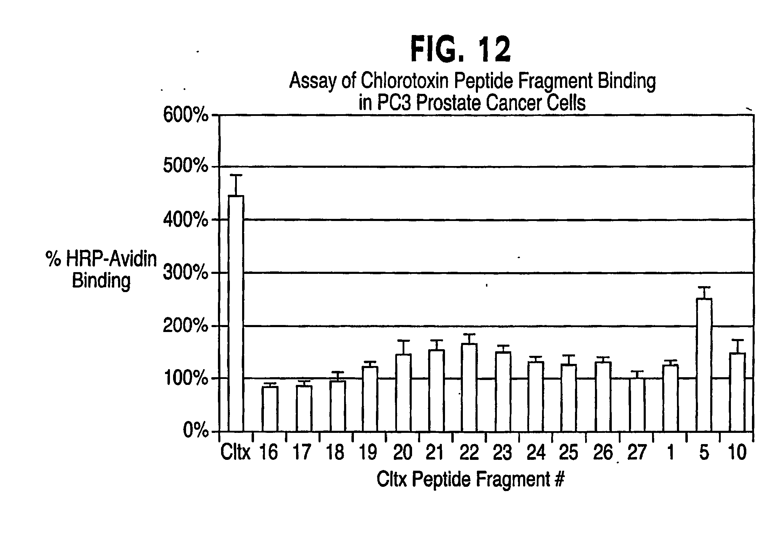

Figure 12 shows that peptide 19 of SEQ ID NO: 1 does not bind PC3 cells but peptide 20 does, indicating that the threonine residue which starts peptide 20 may be the start of a second binding site because peptides 20-24 bind most strongly. Binding decreases again in peptide 25, suggesting that the peptide 24 terminal arginine residue is another key, since this is present in peptide 24 but lost in peptide 25. This indicates that a second binding region of chlorotoxin resides within the 9-mer sequence TDHQMAR (SEQ ID NO: 9) residing at amino acid residues 8-14 of SEQ ID NO: 1 which are common to peptides 20-24. The binding in this second core sequence is broader, which may be a reflection of very similar amino acids present at the ends of the region. For example, there are two threonine residues at peptides 20 and 21, and there is a lysine at the end of peptide 22 next to the arginine residue.

### Example 12

To determine the *in vivo* activity of these identified binding regions, 10-mer peptides 5 (amino acid residues 23-32), 12 (amino acid residues 16-25; as a negative control) and 21 (amino acid residues 7-16) of SEQ ID NO: 1 were used in a crayfish paralysis assay, an assay which is commonly used to determine the bio-activity of chlorotoxin (see DeBin et al. (1993) Am. J. Physiol. 264, C361-369). Peptides 5 and 12 failed to paralyze crayfish, while peptide 21 was effective, indicating that the site which is responsible for the paralytic effect of chlorotoxin is the region defined by peptide 21.

Additionally, several of the chlorotoxin derivatives were each analyzed in the crayfish assay and compared to chlorotoxin (Table 4). Each of these derivatives comprises the putative end-amino acids, the T and the R within the sequence corresponding to peptide 21.

| **Table 4** | | | | |
|---|---|---|---|---|
| **Peptide** | **SEQ ID** | **Crayfish Assay** | **Identity** | **Sequence Comparison** |
| Cltx | 1 | Yes | 100% | TDHQMAR (SEQ ID NO: 9) |
| Cltx (Y/F) | 5 | Yes | 100% | TDHQMAR (SEQ ID NO: 9) |
| STP-1 | 6 | Yes | 71.4 % | TPPQMSR (SEQ ID NO: 77) |
| 6xH-Cltx | 2 | Yes | 100% | TDHQMAR (SEQ ID NO: 9) |
| Y-Cltx | 3 | Yes | 100% | TDHQMAR (SEQ ID NO: 9) |
| YSY-Cltx | 4 | Yes | 100% | TDHQMAR (SEQ ID NO: 9) |

### Example 13

Chlorotoxin is a 36-amino acid peptide with 8 cysteines, depicted below in bold type with the sequences of peptide number 8 (beta-region peptide) and peptide number 21 (alpha-region peptide) identified using the overlapping 10-mers in Example 12 underlined below:
MCMPCFTTDHQMARKCDDCCGGKGRCKCYGPQCLCR (SEQ ID NO: 1)

In order to confirm the identify the minimal binding sequences within the alpha and beta peptides, the entire peptides were synthesized as a 10-mer with a biotin at the amino terminus as well as shorter sequences reducing the size of the peptide by one amino acid at the amino terminus each time.

For the beta peptide, the sequences of peptide 8 noted in Table 5 were evaluated and probed for binding to U251 glioma cells:

| | |
|---|---|
| **Table 5** | |

| **Peptide** | **Sequence** |
|---|---|
| 8 | Biotin-GGKGRGKSYG (SEQ ID NO: 78) |
| 8a | Biotin-GKGRGKSYG (SEQ ID NO: 79) |
| 8b | Biotin-KGRGKSYG (SEQ ID NO: 80) |
| 8c | Biotin-GRGKSYG (SEQ ID NO: 81) |

For the alpha peptide, the sequences of peptide 21 noted in Table 6 were evaluated and probed for binding to U251 glioma cells:

| **Table 6** | |
|---|---|
| **Peptide** | **Sequence** |
| 21 | Biotin-TTDHQMARKS (SEQ ID NO: 82) |
| 21a | Biotin-TDHQMARKS (SEQ ID NO: 10) |
| 21b | Biotin-DHQMARKS (SEQ ID NO: 83) |
| 21c | Biotin-HQMARKS (SEQ ID NO: 84) |
| 21d | Biotin-QMARKS (SEQ ID NO: 85) |

Results demonstrated that the initial threonine residue of the alpha-region peptide is detrimental to binding but that the second threonine is crucial to binding. It was also discovered that none of the smaller peptides exhibit binding as strong as the 9-mer of peptide 21a.

### Example 14

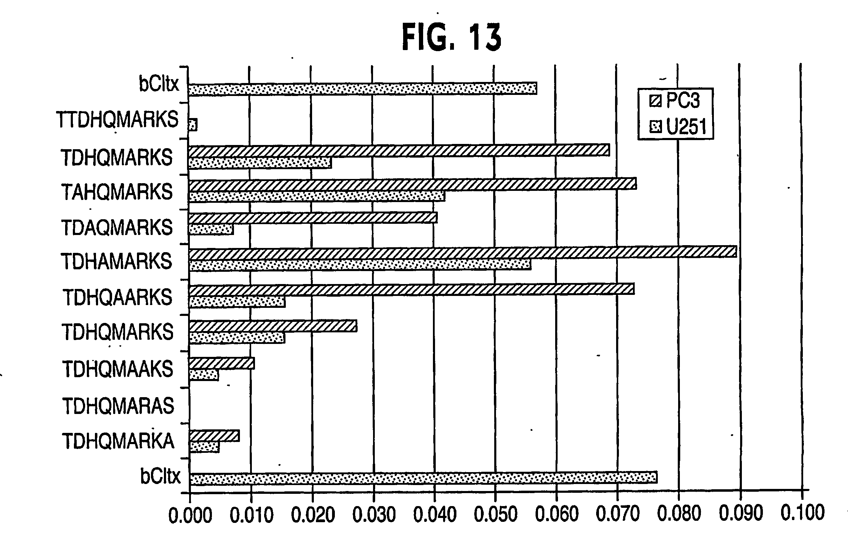

To determine the contribution of each residue to the binding properties of the alpha peptide, alanine scan variants were synthesized by replacing each amino acid of the 9-mer peptide TDHQMARKS (SEQ ID NO: 10) sequentially as depicted in Table 7. Peptide 21, the native core 9-mer, and each alanine-substituted 9-mer peptide was synthesized with a biotin at the amino terminus and evaluated for their binding versus both U251 and PC3 cells (Figure 13).

| **Table 7** | |
|---|---|
| **Peptide** | **Sequence** |
| 21 | Biotin-TTDHQMARKS (SEQ ID NO: 82) |
| 21a | Biotin-TDHQMARKS (SEQ ID NO: 10) |
| 21a-A1 | Biotin-ADHQMARKS (SEQ ID NO: 86) |
| 21a-A2 | Biotin-TAHQMARKS (SEQ ID NO: 87) |
| 21a-A3 | Biotin-TDAQMARKS (SEQ ID NO: 88) |
| 21a-A4 | Biotin-TDHAMARKS (SEQ ID NO: 89) |
| 21a-A5 | Biotin-TDHQAARKS (SEQ ID NO: 90) |
| 21a-A6 | Biotin-TDHQMARKS (SEQ ID NO: 10) |
| 21a-A7 | Biotin-TDHQMAAKS (SEQ ID NO: 91) |
| 21a-A8 | Biotin-TDHQMARAS (SEQ ID NO: 92) |
| 21a-A9 | Biotin-TDHQMARKA (SEQ ID NO: 93) |

The pattern for U251 and PC3 binding are generally similar. Replacement of the aspartic acid (D) residue in the second position of the 9-mer increased binding of the peptide to cells and replacement of the Q residue in the fourth position produced a large increase of peptide binding to cells. Accordingly, the peptide TAHAMARKS (SEQ ID NO: 11) should be more active than the parent peptide TDHQMARKS (SEQ ID NO: 10). Based on the binding of the peptide TDHAMARKS, this binding may be equal to or greater than chlorotoxin itself.

Based upon this finding, it is expected that a variant peptide of chlorotoxin of the sequence below may be stronger in binding than the native chlorotoxin polypeptide.
MCMPCFTTAHAMARKCDDCCGGKGRCKCYGPQCLCR (SEQ ID NO: 12)

### Example 15

In order to compare binding of the short scorpion toxins, the regions homologous to peptide 21 of small toxin and probable toxin LQH-8/6 were synthesized and biotinylated for analysis in the chlorotoxin binding assay (see Table 8 for amino acid sequences of the peptides).

| **Table 8** | |
|---|---|
| **Scorpion Toxin** | **Peptide 21** |
| Chlorotoxin | TTDHQMARKS (SEQ ID NO: 82) |
| Small Toxin | TTDPQMSKK (SEQ ID NO: 94) |
| Probable Toxin LQH-8/6 | TTDQQMTKK (SEQ ID NO: 95) |

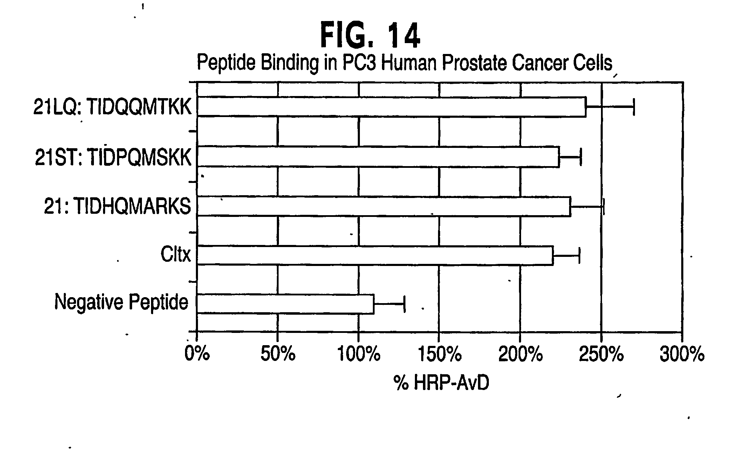

As shown in Figure 14, and in accordance with previous results, chlorotoxin exhibited significant binding in PC3 human prostate cancer cells (221.93% of background levels) and peptide 21 binding paralleled that of chlorotoxin (232.50% of background levels). Additionally, peptide 21 of small toxin peptide (21ST) and peptide 21 of probable toxin LQH-8/6 (21LQ) demonstrated binding levels equivalent to that of full-length chlorotoxin and chlorotoxin peptide 21 (225.26% and 242.32%, respectively). Furthermore, a negative peptide containing amino acids 26-35 of chlorotoxin (SEQ ID NO: 1) exhibited binding levels comparable to background (110%). Similar results were obtained in D54 glioblastoma cells (data not shown).

The results from this study using the chlorotoxin binding assay indicate that chlorotoxin, small toxin peptide, and probable toxin LQH-8/6 bind similarly to human cancer cells *in vitro.* Table 9 below highlights amino acids conserved within the putative primary binding domain (amino acids 7-16) of the three toxin peptides.

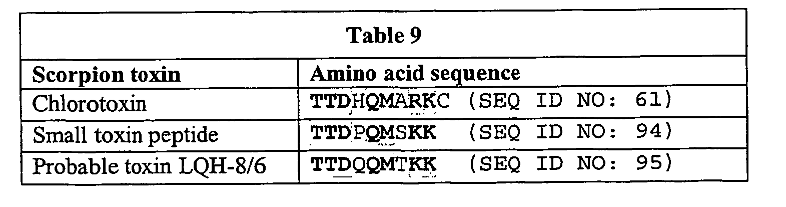

### Example 16

The purpose of this experiment was to determine if the proliferation D54MG Glioblastoma cells, as measured by ³H-thymidine uptake, is effected by Peptide 21, a segment of the full chlorotoxin sequence. The sequence of peptide 21 and its relation to chlorotoxin is shown in the sequence below:
Chlorotoxin: MCMPCFTTDHQMARKCDDCCGGKGRGKCYGPQCLCR
Peptide 21: TTDHQMARK (SEQ ID NO: 82)
Peptide 21 (SEQ ID NO: 82) has been identified in several other reports as having binding and biological activity comparable to the full length chlorotoxin.

D54MG cells were plated in a 24 well plate at 100,000 cells/ml/well using five rows of four wells for each concentration. The cells were allowed to adhere in normal media for twenty-four hours at 37°C and 5% carbon dioxide. TM-701 was diluted to a 1 nM stock solution and added to each row at the concentrations of 0, 20, 80, 160 and 320 nM.

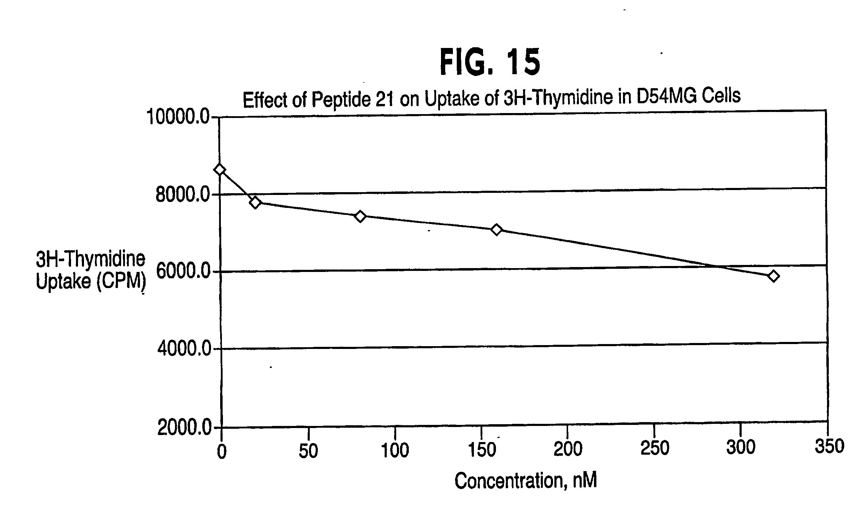

The cells and peptide 21 were allowed to incubate for 24 hours at 37°C and 5% carbon dioxide. After twenty-four hours, the cells were rinsed two times with warm PBS. Normal media was added back to the cells at 1 ml/well. One µCi of ³H-thymidine was added to each well (1 µl of 1 mCi/ml ³H-thymidine to each well). The plate was incubated for two hours at 37°C. The media and thymidine were removed and the wells were rinsed with ice-cold phosphate-buffered saline three times. To each well was added 1 ml of 0.3 N NaOH. The plate was incubated in the 37°C incubator for thirty minutes. Each well of 0.3 N NaOH was pipetted up and down three to four times and removed from the plate and the solution was placed in scintillation vials for counting. Scintillation fluid at four times the amount of sample was added to the vials (4 ml). Each vial was counted on the scintillation counter for one minute. The results are shown in Table 10 and Figure 15. The data demonstrates that peptide 21 behaves similar to chlorotoxin, in that the uptake of ³H-thymidine decreases in a does-dependent manner. This data also indicates that peptide 21 has an effect on the DNA synthesis in these cells.

| **Table 10** | | |
|---|---|---|
| [Peptide 21] (nM) | ³H-Thymidine uptake ± SD (CPM) | |
| 0 | 8645 ± 1218 | 1218 |
| 20 | 7795 ± 634 | 634 |
| 80 | 7412 ± 630 | 630 |
| 160 | 6983 ± 329 | 329 |
| 320 | 5782 ± 886 | 886 |

## Claims

1. A combination comprising (i) chlorotoxin or a chlorotoxin derivative and (ii) temozolomide, for use in the treatment of a neuroectodermal or prostate cancer,
wherein chlorotoxin comprises the sequence set forth in SEQ ID NO: 1, and
wherein the chlorotoxin derivative comprises the sequence TTX₁X₂X₃MX₄X₅K (SEQ ID NO: 13), wherein
(i) X₁ is an acidic amino selected from the group consisting of aspartic acid and glutamic acid;
(ii) X₂ is an amino acid selected from the group consisting of alanine, arginine, asparagine, aspartic acid, cysteine, glutamine, glutamic acid, glycine, histidine, isoleucine, leucine, lysine, proline, methionine, phenylalanine, serine, threonine, tryptophan, tyrosine and valine;
(iii) X₃ is an amide amino acid selected from the group consisting of asparagine and glutamine;
(iv) X₄ is an amino acid selected from the group consisting of serine, threonine and alanine; and
(v) X₅ is a basic amino acid selected from the group consisting of histidine, lysine and arginine.

2. A combination according to claim 1, wherein the chlorotoxin or chlorotoxin derivative is to be administered simultaneously with temozolomide.

3. A combination according to claim 1, wherein the chlorotoxin or chlorotoxin derivative is to be administered prior to the administration of temozolomide.

4. A combination according to any one of claims 1 to 3, wherein the cancer is selected from neoplasms of the central nervous system (CNS), spinal axis tumors, glioma, meningioma and pituitary adenoma.

5. The use of (i) chlorotoxin or a chlorotoxin derivative and (ii) temozolomide for the manufacture of a medicament for use in the treatment of a neuroectodermal or prostate cancer,
wherein chlorotoxin comprises the sequence set forth in SEQ ID NO: 1, and
wherein the chlorotoxin derivative comprises the sequence TTX₁X₂X₃MX₄X₅K (SEQ ID NO: 13), wherein
(i) X₁ is an acidic amino selected from the group consisting of aspartic acid and glutamic acid;
(ii) X₂ is an amino acid selected from the group consisting of alanine, arginine, asparagine, aspartic acid, cysteine, glutamine, glutamic acid, glycine, histidine, isoleucine, leucine, lysine, proline, methionine, phenylalanine, serine, threonine, tryptophan, tyrosine and valine;
(iii) X₃ is an amide amino acid selected from the group consisting of asparagine and glutamine;
(iv) X₄ is an amino acid selected from the group consisting of serine, threonine and alanine; and
(v) X₅ is a basic amino acid selected from the group consisting of histidine, lysine and arginine.

6. The use of claim 5, wherein the chlorotoxin or chlorotoxin derivative is to be administered simultaneously with temozolomide.

7. The use of claim 5, wherein the chlorotoxin or chlorotoxin derivative is to be administered prior to the administration of temozolomide.

8. The use of any one of claims 5 to 7, wherein the cancer is selected from neoplasms of the central nervous system (CNS), spinal axis tumors, glioma, meningioma and pituitary adenoma.

## Patentansprüche

1. Kombination umfassend (i) Chlorotoxin oder ein Chlorotoxinderivat und (ii) Temozolomid für die Verwendung bei der Behandlung eines neuroektodermalen Tumors oder von Prostatakrebs,
wobei das Chlorotoxin die Sequenz dargelegt in SEQ ID Nr. : 1 umfasst, und
wobei das Chlorotoxinderivat die Sequenz TTX₁X₂X₃MX₄X₅K (SEQ ID Nr.: 13) umfasst, wobei
(i) X₁ eine saure Aminosäure ist, die aus der Gruppe bestehend aus Asparginsäure und Glutaminsäure gewählt wird;
(ii) X₂ eine Aminosäure ausgewählt aus der Gruppe bestehend aus Alanin, Arginin, Asparagin, Asparginsäure, Cystein, Glutamin, Glutaminsäure, Glycin, Histidin, Isoleucin, Leucin, Lysin, Prolin, Methionin, Phenylalanin, Serin, Threonin, Tryptophan, Tyrosin und Valin ist;
(iii) X₃ eine Amid-Aminosäure ausgewählt aus der Gruppe bestehend aus Asparagin und Glutamin ist;
(iv) X₄ eine Aminosäure ausgewählt aus der Gruppe bestehend aus Serin, Threonin und Alanin ist; und
(v) X₅ eine basische Aminosäure ausgewählt aus der Gruppe bestehend aus Histidin, Lysin und Arginin ist.

2. Kombination gemäß Anspruch 1, wobei das Chlorotoxin oder Chlorotoxinderivat gleichzeitig mit Temozolomid zu verabreichen ist.

3. Kombination gemäß Anspruch 1, wobei das Chlorotoxin oder Chlorotoxinderivat vor der Verabreichung des Temozolomids zu verabreichen ist.

4. Kombination gemäß einem der Ansprüche 1 bis 3, wobei der Tumor aus Neoplasma des zentralen Nervensystems (ZNS), Spinalachsentumoren, Gliom, Meningiom und Hypophysenadenom selektiert wird.

5. Verwendung von (i) Chlorotoxin oder eines Chlorotoxinderivats und (ii) Temozolomids für die Herstellung eines Medikaments für die Verwendung bei der Behandlung eines neuroektodermalen Tumors oder von Prostatakrebs,
wobei das Chlorotoxin die Sequenz dargelegt in SEQ ID Nr: 1 umfasst, und
wobei das Chlorotoxinderivat die Sequenz TTX₁X₂X₃MX₄X₅K (SEQ ID Nr. 13) umfasst, wobei
(i) X₁ eine saure Aminosäure ist, die aus der Gruppe bestehend aus Asparginsäure und Glutaminsäure gewählt wird;
(ii) X₂ eine Aminosäure ausgewählt aus der Gruppe bestehend aus Alanin, Arginin, Asparagin, Asparginsäure, Cystein, Glutamin, Glutaminsäure, Glycin, Histidin, Isoleucin, Leucin, Lysin, Prolin, Methionin, Phenylalanin, Serin, Threonin, Tryptophan, Tyrosin und Valin ist;
(iii) X₃ eine Amid-Aminosäure ausgewählt aus der Gruppe bestehend aus Asparagin und Glutamin ist;
(iv) X₄ eine Aminosäure ausgewählt aus der Gruppe bestehend aus Serin, Threonin und Alanin ist; und
(v) X₅ eine basische Aminosäure ausgewählt aus der Gruppe bestehend aus Histidin, Lysin und Arginin ist.

6. Verwendung von Anspruch 5, wobei das Chlorotoxin oder Chlorotoxinderivat gleichzeitig mit Temozolomid zu verabreichen ist.

7. Verwendung von Anspruch 5, wobei das Chlorotoxin oder Chlorotoxinderivat vor der Verabreichung des Temozolomids zu verabreichen ist.

8. Verwendung eines der Ansprüche 5 bis 7, wobei der Tumor aus Neoplasma des zentralen Nervensystems (ZNS), Spinalachsentumoren, Gliom, Meningiom und Hypophysenadenom selektiert wird.

## Revendications

1. Combinaison comprenant (i) de la chlorotoxine ou un dérivé de chlorotoxine et (ii) du témozolomide, en vue d'une utilisation dans le traitement d'un cancer neuro-ectodermique ou de la prostate,
dans laquelle la chlorotoxine est composée de la séquence représentée par SEQ ID NO : 1, et
dans laquelle le dérivé de chlorotoxine est composé de la séquence TTX₁X₂X₃MX₄X₅K (SEQ ID NO : 13), dans laquelle
(i) X₁ représente un acide aminé choisi dans le groupe constitué d'acide aspartique et acide glutamique ;
(ii) X₂ représente un acide aminé choisi dans le groupe constitué d'alanine, arginine, asparagine, acide aspartique, cystéine, glutamine, acide glutamique, glycine, histidine, isoleucine, leucine, lysine, proline, méthionine, phénylalanine, sérine, thréonine, tryptophane, tyrosine et valine ;
(iii) X₃ représente un acide aminé amidé choisi dans le groupe constitué d'asparagine et glutamine ;
(iv) X₄ représente un acide aminé choisi dans le groupe constitué de sérine, thréonine et alanine ; et
(v) X₅ représente un acide aminé basique choisi dans le groupe constitué d'histidine, lysine et arginine.

2. Combinaison selon la revendication 1, dans laquelle la chlorotoxine ou le dérivé de chlorotoxine doit être administré simultanément avec du témozolomide.

3. Combinaison selon la revendication 1, dans laquelle la chlorotoxine ou le dérivé de chlorotoxine doit être administré avant l'administration du témozolomide.

4. Combinaison selon l'une quelconque des revendications 1 à 3, dans laquelle le cancer est choisi parmi des néoplasmes du système nerveux central (SNC), des tumeurs de l'axe spinal, un gliome, un méningiome et un adénome hypophysaire.

5. Utilisation (i) de chlorotoxine ou d'un dérivé de chlorotoxine et (ii) de témozolomide pour la fabrication d'un médicament destiné à une utilisation dans le traitement d'un cancer neuro-ectodermique ou de la prostate,
dans laquelle la chlorotoxine est composée de la séquence représentée par SEQ ID NO : 1, et
dans laquelle le dérivé de chlorotoxine est composé de la séquence TTX₁X₂X₃MX₄X₅K (SEQ ID NO : 13), dans laquelle
(i) X₁ représente un acide aminé choisi dans le groupe constitué d'acide aspartique et acide glutamique ;
(ii) X₂ représente un acide aminé choisi dans le groupe constitué d'alanine, arginine, asparagine, acide aspartique, cystéine, glutamine, acide glutamique, glycine, histidine, isoleucine, leucine, lysine, proline, méthionine, phénylalanine, sérine, thréonine, tryptophane, tyrosine et valine ;
(iii) X₃ représente un acide aminé amidé choisi dans le groupe constitué d'asparagine et glutamine ;
(iv) X₄ représente un acide aminé choisi dans le groupe constitué de sérine, thréonine et alanine ; et
(v) X₅ représente un acide aminé basique choisi dans le groupe constitué d'histidine, lysine et arginine.

6. Utilisation selon la revendication 5, dans laquelle la chlorotoxine ou le dérivé de chlorotoxine doit être administré simultanément avec du témozolomide.

7. Utilisation selon la revendication 5, dans laquelle la chlorotoxine ou le dérivé de chlorotoxine doit être administré avant l'administration du témozolomide.

8. Utilisation selon l'une quelconque des revendications 5 à 7, dans laquelle le cancer est choisi parmi des néoplasmes du système nerveux central (SNC), des tumeurs de l'axe spinal, un gliome, un méningiome et un adénome hypophysaire.
